# EUROPÄISCHE PATENTSCHRIFT

(11) **EP 0 762 904 B1**
(45) Veröffentlichungstag und Bekanntmachung des Hinweises auf die Patenterteilung: **02.10.2002**
(21) Anmeldenummer: 95921770.4
(22) Anmeldetag: 29.05.1995
(51) Int. Cl.: A61M 5/20, A61M 5/24

(54) **INJEKTIONSGERÄT**
INJECTION DEVICE
DISPOSITIF D'INJECTION

(30) Priorität: 30.05.1994 DE 4418824
(43) Veröffentlichungstag der Anmeldung: 19.03.1997
(60) Teilanmeldung: 02008599.9
(73) Patentinhaber: B D Medico S.a.r.l., 1295 Mies (CH)
(72) Erfinder: BECHTOLD, Herbert, D-71139 Ehningen (DE); GABRIEL, Jochen, D-70192 Stuttgart (DE)
(74) Vertreter: Raible, Hans, Dipl.-Ing.
(86) Internationale Anmeldenummer: EP9502032
(87) Internationale Veröffentlichungsnummer: WO95032749

(56) Entgegenhaltungen:
- EP-A- 0 268 191
- WO-A-87/02895
- WO-A-91/10460
- WO-A-94/17846
- DE-B- 2 056 688
- US-A- 2 695 023
- US-A- 4 659 327
- US-A- 4 973 318

## Beschreibung

Die Erfindung betrifft ein Injektionsgerät, welches zur Abgabe mehrerer Injektionsdosen geeignet ist. Solche Injektionsgeräte werden z.B. von Patienten mit perniziöser Anämie verwendet, um sich regelmäßig Injektionen mit Hydroxycobalamin zu geben.

Bei solchen Geräten, die von Laien verwendet werden, ist es wichtig, dass die Bedienung einfach und leicht verständlich ist, und dass die Injektion wenig Schmerzen verursacht. Auch ist es wünschenswert, dass der Patient, der besonders vor der Nadel Angst hat, diese nicht direkt sieht, und dass die Reproduzierbarkeit der eingestellten Injektionsdosis gut ist.

Halb- und vollautomatische Injektionsgeräte sind in großer Vielzahl bekannt. Ihr Aufbau ist oft sehr kompliziert, so dass die Herstellkosten hoch sind. Soll ein Gerät nach Gebrauch nicht wiederverwendet, sondern weggeworfen oder dem Recycling zugeführt werden, so muss ein solches Gerät aus wenigen Teilen und sehr preiswert herstellbar sein. Die meisten Injektionsgeräte, welche halb- oder vollautomatisch arbeiten, genügen dieser Forderung nicht. Hier wird z.B. auf die vollautomatischen Injektionsgeräte nach der DE-A1-3 914 818, der DE-A1-4 013 769, oder dem DE-U-9 200 192 hingewiesen. Diese Geräte benötigen eine erste Feder für das Einstechen der Nadel, und eine zweite Feder für das Injizieren der Flüssigkeit, ferner entsprechende Steuereinrichtungen, die den Injektionsvorgang mittels der zweiten Feder erst auslösen, wenn das Einstechen der Nadel beendet ist, wodurch diese Geräte sehr kompliziert und teuer werden.

Aus der EP-A-0651662 kennt man ein Injektionsgerät, aus welchem mehrere Injektionen abgegeben werden können. Dieses Gerät hat ein Gehäuse, in dem ein Halter zwischen einer distalen Endstellung und einer proximalen Endstellung verschiebbar ist. In dem Halter befindet sich ein Behälter mit Injektionsflüssigkeit, die mittels eines Kolbens aus dem Behälter ausgepresst werden kann. Relativ zum Gehäuse ist ein Stößel verschiebbar. Dieser dient zum Verschieben des Halters in distaler Richtung vor einer Injektion und zum Auspressen von Flüssigkeit aus den Behälter während einer Injektion. Dazu ist zwischen dem Halter und dem Stößel eine Langlochverbindung vorgesehen, mit der der Halter beim Spannen des Geräts in seine distale Endstellung gezogen wird. Bei einer Injektion ist diese Langlochverbindung nicht wirksam, sondern hierbei wirkt der Stößel direkt auf den Kolben im Behälter, wodurch er den hydraulischen Druck im Behälter erhöht und diesen dadurch in proximaler Richtung bewegt, um eine Injektionsnadel in den Patienten einzustechen. Wenn der Behälter bei seiner proximalen Bewegung blockiert wird, z.B. durch eine Verschmutzung im Injektionsgerät, wird eine entsprechend erhöhte Flüssigkeitsmenge injiziert. Ebenso kann bereits vor dem Einstechen ein Flüssigkeitsverlust durch die Injektionsnadel auftreten, wodurch dann zu wenig injiziert wird. Die Dosiergenauigkeit hat also bei diesem Gerät eine sehr schlechte Reproduzierbarkeit, weil zu wenig oder zu viel injiziert werden kann, und weil diese Fehldosierung zufallsabhängig ist. Bei medizinischen Geräten ist dies unzulässig, weil Fehldosierungen zu Gesundheitsschäden führen können.

Ferner kennt man aus der US-A-4 973 318 ein Injektionsgerät mit einer Kartusche, die fest und unverschiebbar im Gehäuse des Injektionsgeräts angeordnet ist. Zur Verschiebung eines Kolbens in dieser Kartusche dient ein Stößel, und diesem ist eine Rücklaufsperre zugeordnet, um zu verhindern, dass der Patient den Stößel in distaler Richtung aus dem betreffenden Gehäuseteil herauszieht, wodurch das Gerät in zwei Teile zerfallen könnte.

Ans der US-A-2 695 023 ist ein Injektionsgerät bekannt, das für die Massenimpfung von Tieren bestimmt ist. Hierzu kann dieses Gerät auf Injektionsdosen von 1 ml, 2 ml, 3 ml, 4 ml oder 5 ml umgestellt werden, und nach jedem Drücken einer Taste kann der Tierarzt die einmal gewählte Dosis durch Druck auf einen Stößel erneut injizieren, bis die Injektionsflüssigkeit verbraucht ist. Im Verlauf der Injektionen ist eine Änderung der Dosis nicht mehr möglich, sondern nur dann, wenn das Injektionsgerät voll gefüllt ist.

Bei diesem Gerät wird auf ein äußeres Rohr ein Kopfteil so weit aufgeschraubt, bis eine Dichtung zusammengepresst ist. Die Länge von Gerät oder Stößel ist jedoch nicht veränderbar.

Aus der US-A-4 659 327 ist ein Gerät bekannt, mit dem ein Zahnarzt Zahnzement aus einer auswechselbaren Kartusche exakt dosieren kann. Bei jedem Druck auf eine Taste wird unter der Wirkung einer vorgespannten Feder eine bestimmte Menge Zement gespendet, z.B. direkt in den Zahn des Patienten. Hierzu dient eine Zahnstange, die zwei Zahnreihen mit gegeneinander versetzten Zähnen hat. Bei jedem Tastendruck bewegt sich diese Zahnstange um eine einzige Zahnteilung weiter.

Es ist eine Aufgabe der Erfindung, ein neues Injektionsgerät bereitzustellen.

Diese Aufgabe wird nach der Erfindung gelöst durch ein Injektionsgerät gemäß Patentanspruch 1. Bei einer distalen Bewegung ist also der Stößel durch die erste Verbindungsanordnung mit dem Behälter gekoppelt, d.h. deren Bewegungen sind "zwangssynchronisiert". Dadurch, dass die erste Verbindungsanordnung auch bei einer Bewegung des Stößels in proximaler Richtung wirksam ist, um während einer vorgegebenen Wegstrecke diese Bewegung über ein während dieser Wegstrecke wirksame mechanische Verbindung zwischen Stößel und Behälter (bzw. zwischen Stößel und Halter) in eine entsprechende Bewegung des Behälters umzusetzen, wird die Reproduzierbarkeit der eingestellten Dosis wesentlich verbessert. Eine ordnungsgemäße proximale Bewegung von Stößel und Behälter verschiebt den letzteren so weit, bis er z.B. seine proximale Endstellung im Gehäuse erreicht hat, wonach die proximale Bewegung des Stößels unabhängig vom Behälter erfolgt, um Flüssigkeit aus dem Behälter auszupressen. Bei der ersten Verbindungsanordnung zwischen Stößel und Behälter handelt es sich also bevorzugt um eine gesteuerte Verbindungsanordnung, d.h. der Behälter folgt der Bewegung des Stößels nur dort, wo das sinnvoll und für die Funktion des Geräts erforderlich ist. Bevorzugt wird die erste Verbindungsanordnung bei der proximalen Bewegung durch die Lage des Behälters relativ zum Gehäuse gesteuert.

Zweckmäßig wird die unidirektionale Zwangsverbindung so ausgebildet, dass ein Anschlag für die distale Bewegung des Behälters auch eine Begrenzung für die distale Bewegung des Stößels bildet. Gelangt also der Behälter in seine distale Endstellung, so wird eine weitere Bewegung des Stößels in distaler Richtung verhindert.

Nach einer weiteren Ausgestaltung der Erfindung ist die erste Verbindungsanordnung in der distalen Endstellung des Behälters sowohl in proximaler wie in distaler Richtung wirksam, in der proximalen Endstellung des Behälters aber nur bei Bewegung des Stößels in distaler Richtung. Dies ergibt eine sichere Funktion bei einfacher Ausgestaltung.

Eine weitere, sehr bevorzugte Ausgestaltung der Erfindung betrifft ein Injektionsgerät, bei welchem ein Antriebsorgan für den Stößel vorgesehen ist, und zwischen dem Antriebsorgan und dem Stößel eine weg- und/oder richtungsabhängig wirksame zweite Verbindungsanordnung vorgesehen ist. Die zweite Verbindungsanordnung ist also bevorzugt eine gesteuerte Verbindungsanordnung, d.h. der Stößel folgt der Bewegung des Antriebsorgans nur dort, wo das sinnvoll und für die Funktion des Geräts erforderlich ist. Bevorzugt wird auch die zweite Verbindungsanordnung durch die Lage des Behälters relativ zum Gehäuse gesteuert.

Dabei wird die zweite Verbindungsanordnung mit großem Vorteil dazu ausgebildet, bei einer Bewegung des Antriebsorgans in proximaler Richtung eine unidirektionale Zwangsverbindung zwischen Antriebsorgan und Stößel herzustellen und letzteren zusammen mit dem Antriebsorgan in proximaler Richtung zu bewegen, und eine Bewegung des Antriebsorgans in distaler Richtung so weit in eine entsprechende Bewegung des Stößels in distaler Richtung umzusetzen, wie der Stößel an einer Bewegung in distaler Richtung nicht gehindert ist. Dies hat sich für die Funktion des Geräts als sehr vorteilhaft erwiesen und ermöglicht in einfacher Weise eine Einstellung der zu injizierenden Dosis durch eine Relativverschiebung zwischen Antriebsorgan und Stößel.

Auch geht man mit Vorteil so vor, dass die zweite Verbindungsanordnung in einem proximalen Stellungsbereich des Behälters eine Verbindung zwischen Antriebsorgan und Stößel sowohl in proximaler wie in distaler Richtung herstellt. Dies ermöglicht es, nach einer Injektion den Behälter in seine distale Endstellung zu bringen.

Eine weitere vorteilhafte Ausgestaltung der zweiten Verbindungsanordnung ist dadurch gekennzeichnet, dass diese in einem distalen Stellungsbereich des Behälters eine in proximaler Richtung wirksame Verbindung zwischen Antriebsorgan und Stößel herstellt, welche eine Verschiebung des Antriebsorgans relativ zum Stößel in distaler Richtung ermöglicht. Dies gestattet es, nach Erreichen der distalen Endstellung des Behälters das Antriebsorgan relativ zum Stößel in distaler Richtung zu verschieben und dadurch eine Injektionsdosis einzustellen, da hierdurch die Gesamtlänge von Antriebsorgan und Stößel zunimmt.

Eine vollautomatische Injektion wird in einfacher Weise ermöglicht bei einem derartigen Injektionsgerät, bei welchem dem Antriebsorgan eine auf dieses in proximaler Richtung wirkende Feder, insbesondere eine aus Kunststoffmaterial hergestellte Feder, und ein Rastorgan zugeordnet sind, welche Feder durch Verschieben des Rastorgans in distaler Richtung spannbar ist, wobei das Rastorgan bei Erreichen einer vorgegebenen Spannstellung einrastet. Die Feder - in ihrem gespannten Zustand - ermöglicht so über die zweite und die erste Verbindungsanordnung einen vollautomatischen Injektionsvorgang. Dabei wird durch die Energie, welche in dieser Feder nach dem Spannen gespeichert ist, zunächst eine mit dem Behälter verbundene Nadel eingestochen, und dann die Flüssigkeit aus dem Behälter ausgepresst und injiziert. Dies gestattet die Herstellung eines sehr einfachen, aus wenigen Teilen bestehenden Vollautomaten. Jedoch kann ein solches Gerät auch manuell betätigt werden, also ohne Feder, wobei der Benutzer dann durch Betätigen eines einzigen Betätigungsgliedes zunächst die Nadel einsticht und dann die Flüssigkeit in der eingestellten Menge injiziert.

Bei einer sehr einfachen Ausgestaltung ist der Stößel mindestens bereichsweise nach Art einer Zahnstange ausgebildet. Eine solche Zahnstange ist einfach und preiswert herzustellen, z.B. aus einem Kunststoff. Dabei werden die Zähne der Zahnstange mit Vorteil so ausgebildet, dass sie relativ zu einem federnden Rastorgan nur eine Bewegung der Zahnstange in proximaler Richtung gestatten. Auch wird dem Stößel mit Vorteil ein Anschlag zugeordnet, welcher zumindest seine proximale Bewegung relativ zum Gehäuse begrenzt. Hierdurch wird dem Benutzer angezeigt, wenn die im Behälter vorhandene Flüssigkeitsmenge verbraucht ist, bzw. es werden in diesem Fall weitere Injektionsabläufe verhindert, bei denen keine Flüssigkeit mehr injiziert werden könnte.

Dem Behälter wird mit Vorteil ein Halter zugeordnet, welcher im Gehäuse axial zwischen einer proximalen und einer distalen Endstellung verschiebbar ist und welcher den Behälter aufnimmt. Dies schützt den Behälter und ermöglicht es, diesen erst am Ende der Fertigung in das Injektionsgerät einzusetzen, wie das für eine sterile Herstellung wünschenswert ist.

Gemäß einer außerordentlich vorteilhaften Ausgestaltung der Erfindung ist die Länge des Halters veränderbar. Dies ermöglicht die notwendigen Justiervorgänge, denn die Behälter sind zwar in ihrer Größe festgelegt, haben aber stets Fertigungstoleranzen, ebenso der in ihnen gewöhnlich angeordnete Kolben, und diese Toleranzen können durch die Veränderung der Länge des Halters in sehr einfacher Weise kompensiert werden. Dazu weist der Halter mit besonderem Vorteil einen proximalen Abschnitt und einen distalen Abschnitt auf, welche durch eine verstellbare Verbindung miteinander verbunden sind, die zum Beispiel mittels einer axialen Kraft eine Änderung und insbesondere eine Verkürzung der Gesamtlänge des Halters ermöglicht, insbesondere durch eine Mikrorastung. Eine solche Mikrorastung, oder beispielsweise eine entsprechende Verschraubung mit einem Feingewinde, erlaubt eine Feineinstellung der Länge des Halters und damit eine sehr exakte Justierung, wie sie für eine exakte Einhaltung der Injektionsdosis wünschenswert ist.

Dabei liegt der Halter mit Vorteil in seiner proximalen Endstellung mit seinem distalen Ende gegen einen proximalen Endabschnitt des Antriebsglieds an. Dies ermöglicht eine einfache Feineinstellung mittels einer axialen Kraft, die z.B. auf das proximale Ende des Halters wirkt und eine Verschiebung einer Mikrorastung bewirkt.

Eine außerordentlich vorteilhafte Weiterbildung der Erfindung betrifft ein Injektionsgerät, bei welchem das Gehäuse mindestens zweiteilig ausgebildet ist, und eine Vorrichtung zur Veränderung der relativen Lage dieser Gehäuseteile, insbesondere durch lineare relative Verschiebung zwischen diesen, vorgesehen ist. Dies ermöglicht in einfacher Weise eine Verstellung der Injektionsdosis, insbesondere, wenn ein erstes Gehäuseteil zur Führung des den Behälter für die Injektionsflüssigkeit aufnehmenden Halters vorgesehen ist und ein zweites, relativ zum ersten verschiebbares Gehäuseteil Rastmittel zur Verrastung eines am Antriebsorgan vorgesehenen Rastorgans aufweist. Da auf diese Weise die Lage der Rastmittel relativ zum Gehäuse verschiebbar ist, ist der Weg veränderbar, den das Antriebsorgan bis zum Einrasten zurücklegt, und dies bedeutet, dass die Injektionsdosis vom Benutzer variiert werden kann, indem er das Teil mit den Rastmitteln relativ zum anderen Gehäuseteil verschiebt.

Eine andere Weiterbildung der Erfindung betrifft ein Injektionsgerät, bei welchem die Zahnreihen auf den einander gegenüberliegenden Seiten einer als Stößel dienenden Zahnstange und/oder die Eingriffselemente auf mit diesen Zahnreihen in federndem Eingriff stehenden Eingriffsgliedern gegeneinander in axialer Richtung versetzt sind, um alternativ einen vollen Eingriff eines Eingriffselements des ersten von zwei federnden Eingriffsgliedern mit der ihm zugeordneten Zahnreihe des Stößels, oder einen vollen Eingriff eines Eingriffselements des zweiten von zwei federnden Eingriffsgliedern mit der ihm zugeordneten Zahnreihe des Stößels, zu ermöglichen, und so eine verfeinerte Einstellmöglichkeit des Injektionsgeräts zu ermöglichen. Dies erweist sich besonders dann von Vorteil, wenn sehr kleine Injektionsdosen eingestellt werden sollen, welche Bewegungen des Kolbens in einer Kartusche entsprechen, die kleiner als 1 mm sind.

Weitere Einzelheiten und vorteilhafte Weiterbildungen der Erfindung ergeben sich aus den im folgenden beschriebenen und in der Zeichnung dargestellten, in keiner Weise als Einschränkung der Erfindung zu verstehenden Ausführungsbeispielen, sowie aus den übrigen Unteransprüchen. Es zeigen:
- Fig. 1 bis 5: Schaubilder, welche den zeitlichen Ablauf bei einem Injektionsvorgang in schematisierter Form widergeben,
- Fig. 6 und 7: eine zeichnerische Erläuterung von Einzelheiten der Fig. 1 bis 5,
- Fig. 8A und 8B: einen Längsschnitt durch eine bevorzugte Ausführungsform eines erfindungsgemäßen Injektionsgeräts, im nicht gespannten Zustand,
- Fig. 9A und 9B: Darstellungen analog Fig. 8, aber im gespannten Zustand,
- Fig. 10: eine schematische raumbildliche Darstellung des proximalen Endes des beim ersten und zweiten Ausführungsbeispiel verwendeten Stößels in Gestalt einer Zahnstange mit etwa quadratischem Querschnitt,
- Fig. 11A, B: einen Längsschnitt analog Fig. 8 und 9, aber in einer zu diesen beiden Figuren senkrechten Ebene,
- Fig. 12: eine Darstellung, welche zeigt, wie ein Haken einer Verbindungsanordnung in den Stößel der Fig. 10 eingreift.
- Fig. 13: eine vergrößerte, schematisierte Darstellung der Rastung zwischen Teilen des Halters 54, wie sie auch beim nachfolgenden dritten Ausführungsbeispiel verwendet wird,
- Fig. 14: eine raumbildliche Darstellung eines Injektionsgeräts nach einem dritten Ausführungsbeispiel der Erfindung, bei welchem eine Verstellung der Injektionsdosis möglich ist,
- Fig. 15: eine raumbildliche Darstellung analog Fig. 14, wobei aber das Gehäuse zur besseren Darstellung teilweise im Schnitt dargestellt ist und im Bereich dieser Schnittdarstellung die inneren Teile des Injektionsgeräts nicht dargestellt sind,
- Fig. 16: eine raumbildliche, auseinandergezogene Darstellung von inneren Teilen des Injektionsgeräts der Fig. 14 und 15,
- Fig. 17: eine raumbildliche, auseinandergezogene Darstellung des Gehäuses der Fig. 14 und 15 und eines in diesem angeordneten Halterteils,
- Fig. 18: eine raumbildliche, auseinandergezogene Darstellung einer Kartusche, ihres Halters, und einer Injektionsnadel,
- Fig. 19 bis 21: verschiedene Darstellungen des Injektionsgeräts nach dem dritten Ausführungsbeispiel im Längsschnitt und bei verschiedenen Stellungen; die Spannfeder ist nicht dargestellt, und bei Fig. 21 ist der Auslöser (Clip), der in diesem Längsschnitt an sich nicht sichtbar wäre, um 45° versetzt gezeichnet,
- Fig. 22: einen Schnitt, gesehen längs der Linie XXII-XXII der Fig. 21,
- Fig. 23: einen Schnitt, gesehen längs der Linie XXIII-XXIII der Fig. 21, wobei im Schnitt nach Fig. 23 die inneren Teile des Injektionsgeräts nicht dargestellt sind,
- Fig. 24: eine Ausschnittsvergrößerung der Stelle XXIV der Fig. 19,
- Fig. 25: eine Ausschnittsvergrößerung der Stelle XXV der Fig. 20,
- Fig. 26: eine stark schematisierte raumbildliche Darstellung des beim dritten Ausführungsbeispiel verwendeten Stößels in Gestalt einer Zahnstange,
- Fig. 27: eine Darstellung, welche nur die obere und die untere Zahnreihe des Stößels der Fig. 26 zeigt,
- Fig. 28: eine Darstellung, welche nur die linke und die rechte Zahnreihe des Stößels der Fig. 26 zeigt,
- Fig. 29: eine schematische Darstellung zur Erläuterung des Vorschubvorgangs bei einem Vorschub um eine halbe Zahnteilung,
- Fig. 30: einen vergrößerten raumbildlichen Ausschnitt aus der in Fig. 31 dargestellten Zahnstange, wobei ein Teil der Zahnstange weggeschnitten dargestellt ist, um die Zahnung besser sichtbar zu machen, und
- Fig. 31: eine raumbildliche, vergrößerte Darstellung einer Zahnstange, wobei ein Teil weggeschnitten dargestellt ist, um die Zahnung besser sichtbar zu machen.

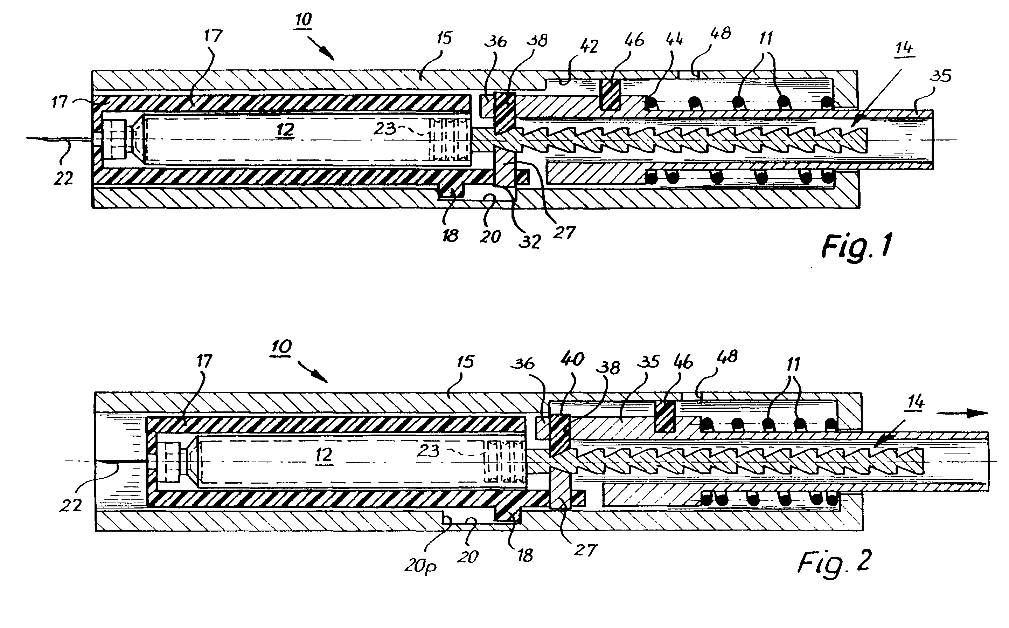

Die Fig. 1 bis 5 zeigen in stark schematisierter Form den Ablauf bei einem Injektionsvorgang.

Fig. 1 zeigt das Injektionsgerät 10 in seiner Ruhelage bei nicht gespannter Feder 11 (analog der Darstellung nach den Fig. 8A und 8B, den Fig. 11A und 11B, sowie den Fig. 19 und 20). Fig. 2 zeigt den ersten Teil des Spannvorgangs der Feder 11, bei welchem ein Behälter (Kartusche) 12 für die zu injizierende Flüssigkeit aus seiner proximalen Endstellung (Fig. 1) in seine distale Endstellung (Fig. 2) gebracht wird, ohne daß hierbei ein Rastorgan 46 bereits in einer vorgegebenen Stellung (Spannstellung) in eine Rastöffnung 48 eingerastet wäre.

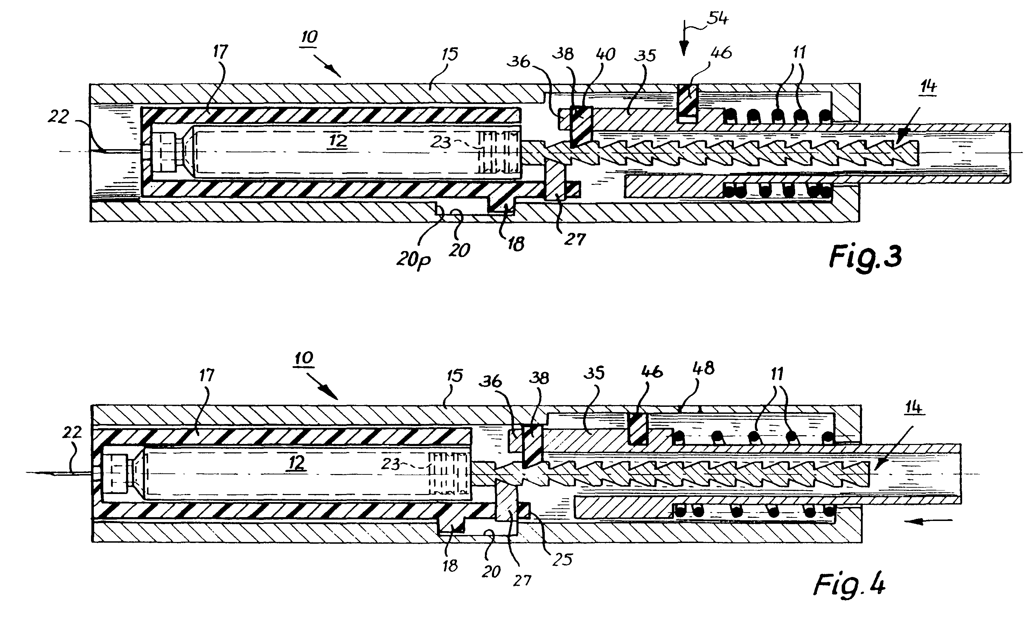

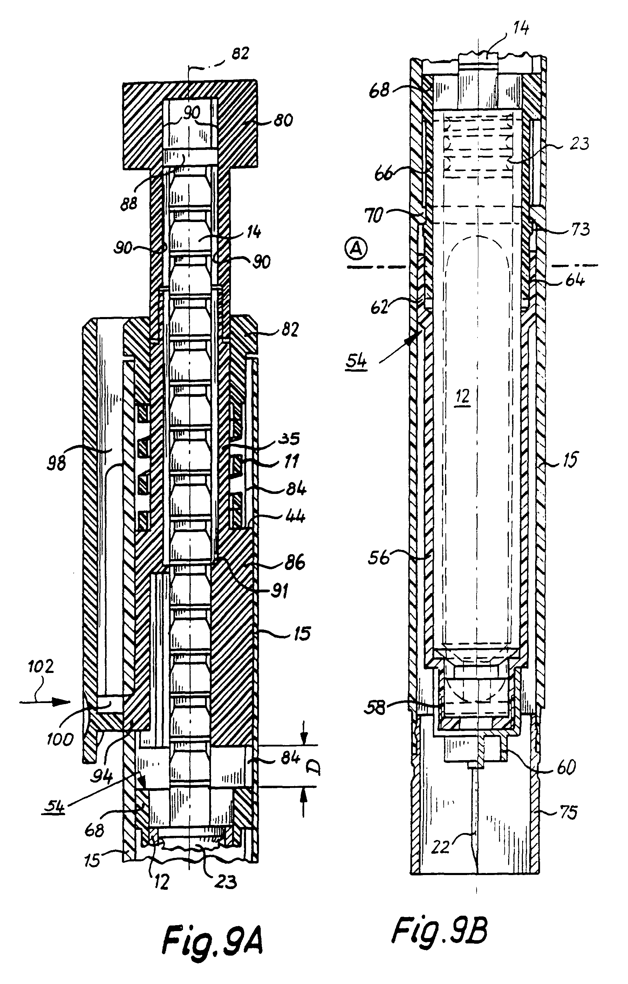

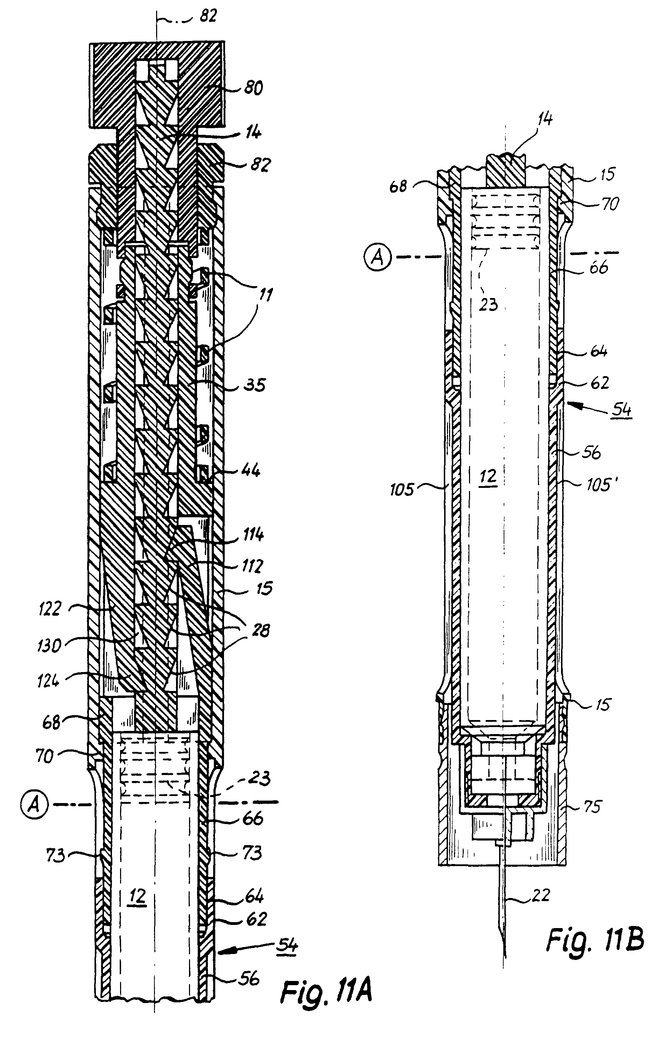

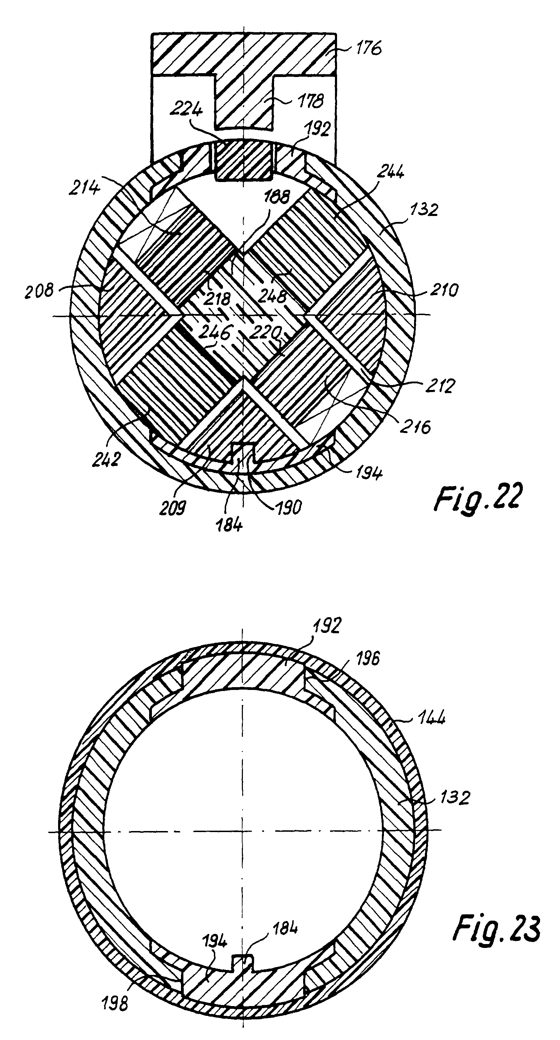

Fig.3 zeigt den Abschluß des Spannvorgangs, bei welchem die Feder 11 voll gespannt und die Injektionsdosis eingestellt ist. Dies entspricht der Darstellung nach den Fig. 9A und 9B, bzw. der Darstellung nach Fig. 21.

Fig. 4 zeigt die erste Phase einer Injektion nach dem Auslösen eines Injektionsvorgangs, wobei die Nadel eingestochen, aber noch keine Flüssigkeit injiziert wird.

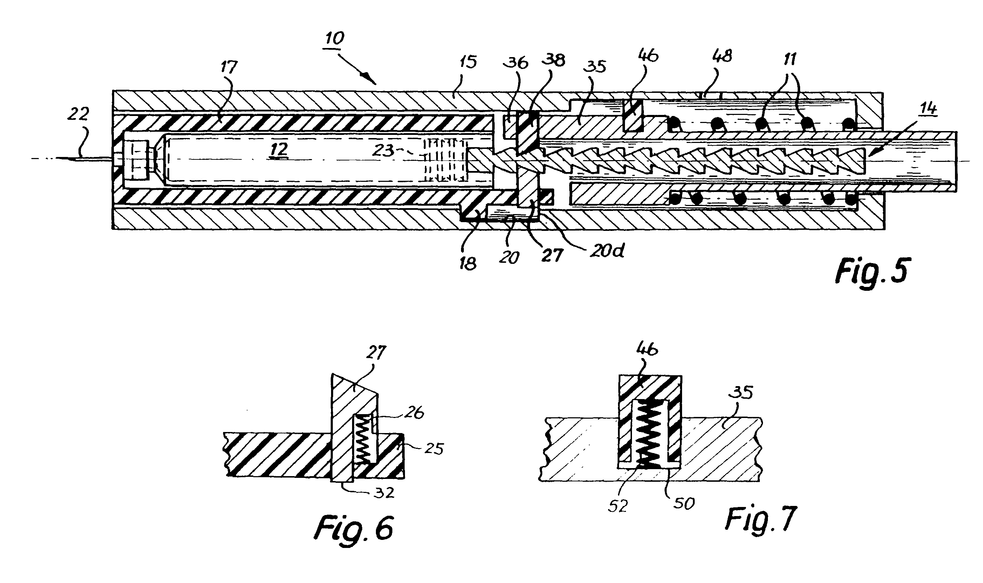

Fig. 5 zeigt die zweite Phase einer Injektion, nach dem Einstechen der Nadel, wobei in Fig. 5 der Zustand nach dem Injizieren der eingestellten Flüssigkeitsmenge dargestellt ist. Das Gerät befindet sich hier wieder in der Ruhelage gemäß Fig. 1, aber der (als Zahnstange ausgebildete) Stößel 14 ist gegenüber Fig. 1 um eine Zahnteilung in proximaler Richtung verschoben, entsprechend der injizierten Flüssigkeitsmenge.

Das Injektionsgerät 10 hat ein Gehäuse 15, in welchem der Behälter 12 in einem Halter 17 zwischen einer proximalen Endstellung (Fig. 1, 4 und 5) und einer distalen Endstellung (Fig. 2 und 3) axial verschiebbar angeordnet ist. Der Halter 17 hat hierzu einen radialen Vorsprung 18, der in einer entsprechenden Ausnehmung 20 des Gehäuses 15 in der dargestellten Weise zwischen zwei axialen Anschlagstellungen verschiebbar ist. Der Behälter 12 selbst ist im Halter 17 in geeigneter Weise fixiert. Der Behälter 12 ist gewöhnlich eine Ampulle aus Glas, an deren proximalem Ende eine Injektionsnadel 22 in der dargestellten, üblichen Weise befestigt werden kann, und in deren distalem Ende ein Kolben 23 verschiebbar angeordnet ist. Solche Ampullen (Kartuschen) werden in großen Stückzahlen produziert. Wird der Kolben 23 in proximaler Richtung verschoben, so wird Flüssigkeit aus dem Behälter 12 durch die Nadel 22 ausgepreßt.

Die Begriffe "proximal" und "distal" sind übliche medizinische Terminologie; sie bedeuten:
Proximal: Zum Patienten hin, also, bezogen auf Fig. 1 bis 5, in Richtung nach links.
Distal: Vom Patienten weg, also bezogen auf die Fig. 1 bis 5, in Richtung nach rechts.

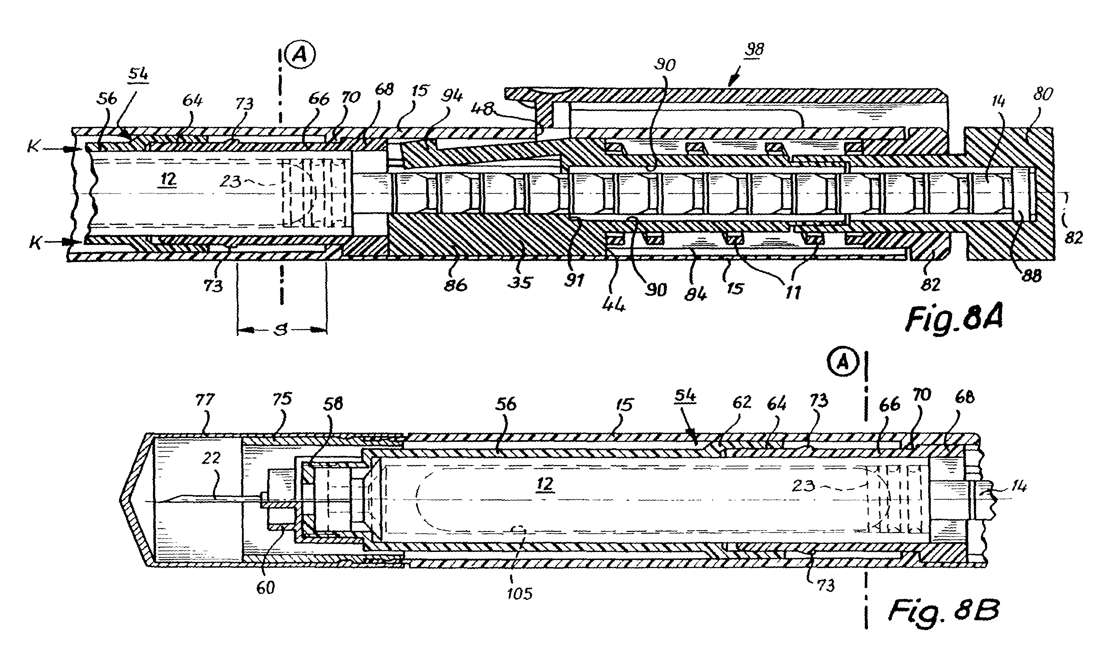

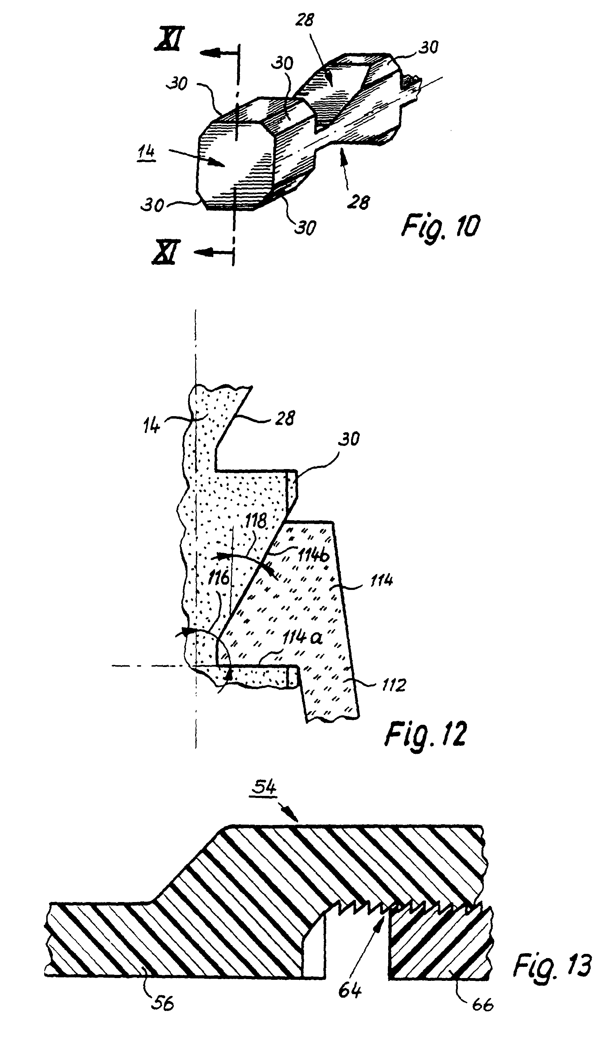

An einem in distaler Richtung sich erstreckenden Fortsatz 25 des Behälters 17 ist - als erste Verbindungsanordnung - ein Rastorgan in Form einer durch eine Feder 26 (Fig. 6) belasteten Klinke 27 angeordnet, und dieses Rastorgan ist so ausgebildet, daß es in eine Lücke der Zahnung 28 (Fig. 10) des Stößels 14 eingreifen kann, dessen bevorzugte raumbildliche Form aus Fig. 10 hervorgeht. Dieser Stößel 14 hat einen im wesentlichen quadratischen Querschnitt mit abgefasten Kanten 30, der auf zwei gegenüberliegenden Seiten mit einer Zahnung 28 in der dargestellten Weise versehen ist, und die Klinke 27 paßt mit ihrer radial inneren Seite in die ihr zugewandte Lücke der Zahnung 28 des Stößels 14.

Die radial äußere Seite 32 (Fig. 6) der Klinke 27 wird gesteuert durch die Form der radialen Innenseite des Gehäuses 15. Bei der Stellung nach den Fig. 1, 4 und 5, also der proximalen Endstetlung des Behälters 12, kann sich diese Klinke 27 radial nach außen bewegen, d.h., wenn der Stößel 14 hier in proximaler Richtung bewegt wird, wird die Klinke 27 radial nach außen verschoben und kann gemäß Fig. 5 in die nächste Zahnlücke 28 gleiten.

Dagegen ist außerhalb der proximalen Endstellung des Behälters 12 die Klinke 27 durch die Innenseite des Gehäuses 15 an einer Bewegung nach außen gehindert, wie das die Fig. 2 und 3 klar zeigen, und so entsteht in diesem Stellungsbereich eine direkte Antriebsverbindung zwischen dem Stößel 14 und dem Halter 17, welche eine Bewegung des Stößels 14 in proximaler Richtung direkt auf den Behälter 12 und die Nadel 22 überträgt. Diese Antriebsverbindung wird erst unterbrochen, wenn der Behälter 12 seine proximale Endstellung (Fig. 1, 4 und 5) erreicht hat, da erst dann die Klinke 27 in der bereits ausführlich beschriebenen Weise radial nach außen verschoben werden kann. Man kann dies auch als Kulissensteuerung oder Nockensteuerung der Klinke 27 bezeichnen.

Hier ist auch darauf hinzuweisen, daß als Rastorgan zum Eingriff in die Lücken der Zahnung 28 viele Varianten möglich sind. Es handelt sich jeweils um federnd auslenkbare Teile, deren federnde Auslenkung außerhalb der proximalen Endstellung gesperrt ist, also um eine wegabhängige Steuerung dieser federnden Auslenkbarkeit, die selbstverständlich auf sehr viele verschiedene Arten möglich ist.

Der Stößel, 14 ist umgeben von einem Antriebsorgan 35, das hier beispielhaft etwa nach Art eine Rohres ausgebildet ist, das um den Stößel 14 herum angeordnet ist und zu dessen Antrieb dient, und zwar über eine zweite Verbindungsanordnung.

Hierzu ist in einem axialen Fortsatz 36 des Antriebsorgans 35 ein Rastorgan in Gestalt einer radial verschiebbaren, federbelasteten Klinke 38 vorgesehen, die genauso aufgebaut ist wie die in Fig. 6 dargestellte Klinke 27, also gegen die Kraft einer Feder radial nach außen verschoben werden kann. Die Klinke 38 greift in einen der Zähne 28 des Stößels 14 ein, und zwar, wie dargestellt, auf der in Fig. 1 oberen Seite, während die Klinke 27 auf der in Fig. 1 unteren Seite in eine der Lücken der Zahnung 28 des Stößels 14 eingreift. (Dies ist der Grund, warum der Stößel 14 auf verschiedenen Seiten mit Zähnen bzw. Zahnlücken versehen ist, bevorzugt auf zwei gegenüberliegenden Seiten.)

Das radial äußere Ende 40 (Fig. 2) der Klinke 38 wird gesteuert von der ihr gegenüberliegenden Innenseite 42 (Fig. 1) des Gehäuses 15. Wie ein Vergleich der Fig. 1 und 2 zeigt, ist eine radiale Bewegung der Klinke 38 im proximalen Endstellungsbereich blockiert, d.h. in diesem Bereich wird eine Bewegung des Antriebsorgans 35 in beiden Richtungen voll auf den Stößel 14 übertragen.

Bei Erreichen der Stellung nach Fig. 2 befindet sich der Halter 17 mit dem Behälter 12 in seiner distalen Endstellung (durch Anschlag des Vorsprungs 18 am distalen Ende 20d der Gehäuseausnehmung 20). Ab dieser Stelle wird die radiale Bewegung der Klinke 38 nicht mehr gesperrt, da ab hier die Innenseite 42 des Gehäuses 15 einen größeren Durchmesser hat. Ab hier kann also die Klinke 38 über einen Zahn des Stößels 14 hinweg in die nächste Zahnlücke gleiten, wie das Fig. 3 zeigt, und dort einrasten.

Da die Spannfeder 11 zwischen dem distalen Ende des Gehäuses 15 und einer Schulter 44 (Fig. 1) des Antriebsorgans 35 angeordnet ist, wird sie bei einer distalen Verschiebung des Antriebsorgans 35 gespannt, und bei Erreichen der Spannstellung greift ein am Antriebsorgan 35 angeordnetes Rastglied 46 in die Ausnehmung 48 des Gehäuses 15 ein und verrastet dort das Antriebsorgan 35 in der Stellung gemäß Fig. 3. Dies ist die Stellung vor einer Injektion. Hierbei befindet sich die Nadel 22 im Gehäuse 15, ist also für den Benutzer nicht sichtbar, was ihm die Angst vor einer Injektion nimmt. Der Halter 17 ist in dieser Stellung im Gehäuse 15 festgehalten, weil er durch die Klinke 27 mit dem Stößel 14 fest verbunden ist, und weil der Stößel 14 seinerseits durch die Klinke 38, die ja mit elastischer Vorspannung gegen den Stößel 14 anliegt, gegen ungewünschte axiale Verschiebungen relativ zum Antriebsorgan 35 gesichert ist, das seinerseits durch das Rastglied 46 im Gehäuse 15 blockiert ist.

Fig. 7 zeigt einen möglichen Aufbau des Rastglieds 46, das hier in einer Ausnehmung 50 des Antriebsorgans 35 radial verschiebbar angeordnet und durch eine Druckfeder 52 radial nach außen beaufschlagt ist. Die Fig. 8 und 9 zeigen eine andere, bevorzugte Ausgestaltung dieses Rastglieds, und diese wird nachfolgend beschrieben werden.

Wird in der Stellung nach Fig. 3 durch Druck auf das Rastglied 46 in Richtung des Pfeiles 54 eine Injektion ausgelöst, so verschiebt die Feder 11 das Antriebsorgan 35 in proximaler Richtung. Über die Klinke 40 wird diese Bewegung direkt auf den Stößel 14 übertragen, und dieser überträgt seinerseits über die Klinke 27, die nicht radial nach außen ausweichen kann, diese Bewegung direkt auf den Halter 17 und den Behälter 12, so daß der Behälter 12 in proximaler Richtung verschoben und die Nadel 22 eingestochen wird, wie das Fig. 4 zeigt.

Fig. 4 zeigt auch, daß dann, wenn der Behälter 12 seine proximale Endstellung erreicht, wobei sein Vorsprung 18 zum Anschlag gegen das proximale Ende 20p der Ausnehmung 20 kommt, die Klinke 27 in den Bereich der Ausnehmung 20 auf der Innenseite des Gehäuses 15 gelangt und folglich jetzt radial nach außen ausweichen kann, d.h. der Stößel 14 kann seine proximale Bewegung fortsetzen und verschiebt jetzt den Kolben 23 im Behälter 12 in proximaler Richtung, und zwar um einen Zahnabstand des Stößels 14, wodurch eine entsprechende Flüssigkeitsmenge aus dem Behälter 12 ausgepreßt und injiziert wird. Der Stößel 14 kann z.B. 10 oder 14 Zähne haben, je nach der Größe der gewünschten Injektionsdosis.

Ist nach mehreren Injektionen die gesamte Flüssigkeit aus dem Behälter 12 ausgepreßt, so ist das Injektionsgerät 10 ausgebraucht und kann anschließend stofflich wiederverwertet werden. Mit einem Stößel 14 mit - wie dargestellt - 14 Zähnen lassen sich also 14 Injektionen mit identischer Dosis verabreichen, und der Vorgang der Injektion läuft nach dem Auslösen automatisch und mit sehr guter Dosiergenauigkeit ab, da die eigentliche Injektion erst beginnt, nachdem die Nadel 22 eingestochen wurde.

Die Fig. 8 bis 13 zeigen eine bevorzugte Ausführungsform der Erfindung. Gleiche oder gleichwirkende Teile wie in den vorhergehenden Figuren werden gewöhnlich mit denselben Bezugszeichen bezeichnet und dann nicht oder nur noch kurz beschrieben.

Wie die Fig. 8B und 13 zeigen, ist der Halter 54 für den Behälter 12 hier zweiteilig ausgebildet. Er hat einen proximalen Abschnitt 56, an dessen proximalem Ende ein Gewinde 58 zum Aufschrauben eines an der Nadel 22 befestigten Kunststoffteils 60 vorgesehen ist, wie das Fig. 8B klar zeigt. Am distalen Ende hat dieser proximale Abschnitt 56 eine Erweiterung 62, die innen mit einer Mikrorastung 64 versehen ist, und in diese greift ein distaler Abschnitt 66, dessen proximales Ende außen ebenfalls mit einer Mikrorastung versehen ist, welche in die Mikrorastung 64 eingreift.
Die Mikrorastung 64 ist in Fig. 13 nur schematisch dargestellt, denn bevorzugt hat sie eine sehr feine Zahnteilung von z.B. 0,1 mm, die zeichnerisch nicht dargestellt werden kann. Die Abschnitte 56 und 66 nehmen in sich den Behälter 12 (mit seinem Kolben 23) in der dargestellten Weise auf. Dies ist ein Standardbehälter, wie er von verschiedenen Firmen hergestellt wird.

Der Aufbau des Halters 54 aus zwei Abschnitten 56 und 66 hat folgenden Grund: Die Behälter 12 und ihre Füllmenge unterliegen den üblichen Schwankungen, so daß bei der Fertigung die Lage des Kolbens 23 relativ zum Halter 54 immer innerhalb gewisser Toleranzgrenzen schwankt.

Es ist aber wichtig, daß bereits vor der ersten Injektion das proximale Ende des Stößels 14 direkt gegen den Kolben 23 anliegt, so, wie in Fig. 8 dargestellt, denn wenn zwischen dem Kolben 23 und dem proximalen Ende des Stößels 14 vor der ersten Injektion ein Spalt vorhanden ist, reduziert sich die erste Injektionsmenge entsprechend, und der Patient erhält zu wenig von der Flüssigkeit, die er injizieren sollte. Deshalb muß ein solcher Spalt vermieden werden.

Dies geschieht dadurch, daß mittels der Mikrorastung 64 der proximale Abschnitt 56 des Halters 54 so lange relativ zum distalen Abschnitt 66 verschoben wird, bis der Kolben 23 ohne Spalt gegen das proximale Ende des Stößels 14 anliegt. Durch die Mikrorastung 64 bleibt anschließend diese Verbindung der Abschnitte 56 und 66 erhalten.

Der distale Abschnitt 66 hat einen Abschnitt 68 größeren Durchmessers, mit dem er in der proximalen Endstellung gegen einen Bund 70 auf der Innenseite des Gehäuses 15 anliegt. Mit diesem Abschnitt 68 ist er auch in einer Längsnut 84 des Gehäuses 15 geführt und gegen Verdrehung gesichert.

Ferner hat der distale Abschnitt 66 auf seiner Außenseite einen Anschlag 73, mit dem er in der distalen Endstellung, welche in Fig. 9B dargestellt ist, gegen den Bund 70 anliegt. Die axiale Bewegung des Halters 54 erfolgt, wie bei den Fig. 1 bis 5 beschrieben, durch die stellungsabhängige Antriebsverbindung mit dem Stößel 14, die nachfolgend anhand der Fig. 11A und 11B beschrieben wird. Der Gesamthub S des Halters 54 ist in Fig. 8A zur Verdeutlichung angegeben.

Das Gehäuse 15 ist bei Fig. 8B mit einer Hülse 75 für die Stichtiefeneinstellung versehen, und auf diese Hülse kann in der dargestellten Weise eine Schutzkappe 77 aufgesetzt werden, um die Nadel 22 zu schützen. Wie Fig. 9B zeigt, befindet sich in der gespannten Stellung die Nadel 22 innerhalb der Hülse 75.

Es ist ferner darauf hinzuweisen, daß in der Ruhelage das Antriebsorgan 35 mit seinem proximalen Ende, und beaufschlagt durch die Spannfeder 11, gegen das distale Ende des Halters 54 anliegt, wie das in Fig. 8A dargestellt ist. (In Fig. 8B ist dies nicht dargestellt.) Dies ermöglicht es, bei der erwähnten Einstellung der Mikrorastung 64 mit einer Kraft K (links in Fig. 8A) in distaler Richtung auf den proximalen Abschnitt 56 des Halters 54 zu drücken und dadurch den Spalt zwischen dem Kolben 23 und dem proximalen Ende des Stößels 14 auf Null zu reduzieren.

Man erkennt auch in Fig. 9A, daß dort ein Abstand D zwischen dem distalen Ende des Halters 54 und dem proximalen Ende des Antriebsorgans 35 liegt, und dieser Abstand D entspricht der zu injizierenden Flüssigkeitsmenge, also hier dem Abstand zwischen zwei Zähnen 28.

Das Antriebsorgan 35 ist bei der Ausführungsform nach den Fig. 8 bis 13 mit einem Ziehknopf 80 zum Spannen der Feder 11 versehen, welcher in der dargestellten Weise mit dem Antriebsorgan 35 fest verbunden ist und mit diesem eine Einheit bildet. (Dieser Knopf ist in den Fig. 1 bis 5 nicht dargestellt.)

Die Längsachse des Injektionsgeräts 10 ist in den Fig. 8 und 9 mit 82 bezeichnet. Es ist darauf hinzuweisen, daß die Darstellungen der Fig. 8 und 9 sehr stark vergrößert sind, d.h. das gesamte Gerät hat etwa die Größe eines überdimensionierten Füllfederhalters mit einer Länge von z.B. 16 bis 17 cm.

Die Spannfeder 11 ist bei der Ausführungsform nach den Fig. 8 bis 13 als Spritzgußteil (Schraubenfeder) aus Kunststoff ausgebildet, und sie ist bevorzugt einstückig mit einem Teil 82, welches in der dargestellten Weise den distalen Abschluß des Gehäuses 15 bildet und mit diesem in der dargestellten Weise verbunden ist. Die Feder 11 liegt mit ihrem proximalen Ende gegen die Schulter 44 des Antriebsorgans 35 an. Letzteres ist in einer entsprechenden zylindrischen Ausnehmung des Teils 82 geführt, wie in Fig. 8A und 9A dargestellt. Es ist ferner mit einer radialen Verbreiterung 86 in der Längsnut 84 des Gehäuses 15 geführt, so daß es sich im Gehäuse 15 nicht drehen kann.

Ebenso ist der Stößel 14 mit einer Verbreiterung 88 an seinem distalen Ende in zwei Längsnuten 90 im Inneren des Antriebsgliedes 35 bzw. des mit ihm verbundenen Knopfes 80 geführt. Diese Längsnuten 90 erstrecken sich bis zu einer Stelle 91. Gelangt die Verbreiterung 88 zu dieser Stelle 91, so kann der Stößel 14 nicht mehr weiter aus dem Antriebsglied 35 herausgeschoben werden, und dadurch wird der bei Fig. 3 dargestellte Betätigungsschritt gesperrt, d.h. das Gerät kann nicht mehr in die Raststellung gemäß Fig. 3 gebracht werden. Dadurch erkennt der Benutzer, daß keine weitere Injektion mehr möglich ist.

Zum Zwecke der Verrastung in der Spannstellung ist am Antriebsorgan 35 ein elastisch nach innen auslenkbarer Rasthaken 94 vorgesehen, der in der dargestellten Weise einstückig mit dem Antriebsorgan 35 ausgebildet ist und wie dieses aus einem elastischen Kunststoff ausgebilde ist. In der Stellung nach Fig. 8A liegt er federnd gegen die Innenseite des Gehäuses 15 an, und in der Raststellung gemäß Fig. 9A, in der die Feder 11 gespannt ist, ist er in die Rastausnehmung 48 (Fig. 8A) im Gehäuse 15 eingerastet.

Zum Auslösen des Injektionsgeräts dient ein Clip 98, welcher, der Rastausnehmung 48 gegenüberliegend, mit einem radial nach innen ragenden Vorsprung 100 versehen ist. Wird der Clip 98, der aus elastischem Kunststoff ausgebildet ist, in Richtung des Pfeiles 102 (Fig. 9A) nach innen gedrückt, so drückt er den Rasthaken 94 aus der Rastausnehmung 48 (Fig. 8A) heraus und löst so einen Injektionsvorgang aus, dessen Ablauf anhand der Fig. 4 und 5 bereits ausführlich beschrieben wurde. Auch der Spannvorgang wurde bereits anhand der Fig. 1 bis 3 ausführlich beschrieben.

Der Clip 98 ist bevorzugt einstückig mit dem Teil 82 und der Feder 11 ausgebildet, was die Herstellung sehr vereinfacht. Das Antriebsorgan 35, der Knopf 80, der Stößel 14 und das Gehäuse 15 sind bevorzugt aus demselben Kunststoff hergestellt wie das Teil 82, die Feder 11 und der Clip 98, was die stoffliche Wiederverwertung des Injektionsgeräts außerordentlich vereinfacht, da der ganze distale Teil des Geräts en bloc der Wiederverwertung zugeführt werden kann. Lediglich für den Halter 54 wird ein transparenter Kunststoff benötigt, damit der Inhalt des Behälters 12 von außen sichtbar ist (ein Fenster 105 im Gehäuse 15 ist in Fig. 88 angedeutet). Deshalb muß der Halter 54 getrennt entsorgt werden, ebenso naturgemäß der Behälter 12, der gewöhnlich aus Glas hergestellt wird.

In Fig. 8, 9 und 11 ist zur Erleichterung der Orientierung eine bestimmte Stelle des Injektionsgeräts mit A bezeichnet. Wie man sieht, überlappen sich die Darstellungen jeweils im Mittelbereich.

Die Fig. 11A und 11B zeigen einen Schnitt, gesehen in Richtung der Linie XI-XI der Fig. 10. Das Injektionsgerät befindet sich bei dieser Darstellung in einer Lage gemäß Fig. 8A und 8B, also in der Lage nach einer Injektion, d.h. die Nadel 22 ragt aus der Hülse 75 heraus. Wie Fig. 11B zeigt, ist das Gehäuse 15 im Bereich der Kartusche 12 mit zwei gegenüberliegenden Fenstern 105, 105' versehen, und da die Teile des Halters 54 aus einem transparenten Kunststoff hergestellt sind, ist es möglich, durch diese Fenster zu sehen, wieviel Flüssigkeit noch im Behälter 12 enthalten ist.

Mit dem distalen Abschnitt 66 des Halters 54 ist gemäß Fig. 11A ein federnder Fortsatz 112 verbunden, der in distaler Richtung und unter einem Winkel von z.B. 10° schräg nach innen verläuft und der an seinem freien, distalen Ende einen Haken 114 hat und mit diesem in eine Zahnlücke 28 des Stößels 14 eingreift. Fortsatz 112 und Haken 114 dienen im Zusammenwirken mit den Zahnlücken 28 des Stößels 14 als eine erste Verbindungsanordnung, welche zwischen dem Stößel 14 und dem Halter 54 wirksam ist. Wie man aus Fig. 12 ersieht, verläuft die proximale Seite 114a des Hakens 114 unter einem Winkel 116 zum Stößel 14, und die distale Schrägseite 114b des Hakens 114 verläuft unter einem Winkel 118 zum Stößel 14. Der Winkel 116 liegt in der Größenordnung von 90°, und der Winkel 118 bevorzugt in der Größenordnung von 15 bis 30°. Ein Winkel von 23° hat sich bei Versuchen als günstig erwiesen.

Bewegt sich der Stößel 14 in distaler Richtung, so zieht er den Haken 114, und mit ihm den Halter 54, in distaler Richtung mit, wodurch sich die Stellung des Halters 54 gemäß Fig. 9B ergibt. Hierbei liegt also eine formschlüssige Verbindung zwischen dem Stößel 14 und dem Haken 114 vor.

Bewegt sich - bei einem Injektionsvorgang - der Stößel 14 in proximaler Richtung, so wird der Haken 114, der ja mit seiner Seite 114b mit Vorspannung gegen den Stößel 14 anliegt, ebenfalls in proximaler Richtung verschoben, d.h. es entsteht eine teils form-, teils kraftschlüssige Verbindung zwischen dem Stößel 14 und dem Haken 114, und der Halter 54 wird solange in der proximalen Richtung bewegt, bis die Stellung gemäß Fig. 11B erreicht ist, in welcher der Abschnitt 68 des Halters 54 gegen den Bund 70 anliegt und eine weitere proximale Bewegung des Halters 54 verhindert. Bewegt sich nun der Stößel 14 in proximaler Richtung weiter, so gleitet der Haken 114 aus der Zahnlücke 28 heraus und in die darauffolgende Zahnlücke 28 des Stößels 14, d.h. die erste Verbindungsanordnung wird unterbrochen, nachdem die Nadel 22 eingestochen wurde, damit nun der Stößel 14 den Kolben 23 in proximaler Richtung verschieben kann. Hierdurch wird die eingestellte Flüssigkeitsmenge aus dem Behälter 12 ausgepreßt und in den Patienten injiziert.

Vom Antriebsorgan 35 ragt ein federnder Fortsatz 122 in proximaler Richtung schräg nach innen, und dieser ist an seinem freien, proximalen Ende mit einer an die Zahnung 28 angepaßten Spitze 124 versehen, die - wie dargestellt - in eine Zahnlücke 28 auf der in Fig. 11A linken Seite des Stößels 14 eingreift. Im Zusammenwirken mit den Zahnlücken 28 wirken der Fortsatz 122 und die Spitze 124 als eine zweite Verbindungsanordnung, die zwischen dem Antriebsorgan 35 und dem Stößel 14 wirksam ist.

Auch hier gilt die schematische Darstellung der Fig. 12 hinsichtlich der Winkel und der bevorzugten Werte dieser Winkel, sofern die Zähne bzw. Zahnlücken 28 auf beiden Seiten des Stößels 14 gleich ausgebildet sind. (Diese können bei Bedarf unterschiedliche Formen und unterschiedliche Winkel haben). Günstig ist es, wenn die Teile 114, 124 von entgegengesetzten Seiten federnd, d.h. mit Vorspannung, gegen den Stößel 14 anliegen, da dieser dann nicht nach einer Seite gebogen wird.

Wird der Ziehknopf 80 in distaler Richtung verschoben, so folgt die Spitze 124 dieser Bewegung, und da sie kraftschlüssig mit dem Stößel 14 verbunden ist, verschiebt sie den Stößel 14 in distaler Richtung. Der Stößel 14 zieht seinerseits über den Haken 114 den Halter 54 in distaler Richtung bis zum Anschlag des Anschlagteils 73 gegen den Bund 70. Dabei durchläuft der Halter 54 den in Fig. 8A dargestellten Weg S.

Beim Anschlag des Teils 73 gegen den Bund 70 wird eine weitere distale Bewegung des Halters 54 blockiert, ebenso eine weitere distale Verschiebung des Stößels 14 (durch den Eingriff des Hakens 114 in eine Zahnlücke 28). Wird nun bei der Spannbewegung am Ziehknopf 80 weiter gezogen, so wird die kraftschlüssige Verbindung zwischen der Spitze 124 und der Zahnlücke 28 gelöst, und die Spitze 124 gleitet in die darauffolgende Zahnlücke 130 des Stößels 14. Auf diese Weise wird die nächste Injektionsdosis eingestellt, die dem Abstand zwischen zwei aufeinanderfolgenden Zähnen bzw. Zahnlücken 28 entspricht. Dabei rastet, wie bereits beschrieben, der Rasthaken 94 in die Rastausnehmung 48 ein.

Beim Auslösen des Injektionsgeräts durch Druck auf den Clip 98 (Fig. 9A) erzeugt die Feder 11 eine Kraft in proximaler Richtung auf die Spitze 124, die sich jetzt in der Zahnlücke 130 befindet. Die Spitze 124 verschiebt durch die formschlüssige Verbindung den Stößel 14 in proximaler Richtung, und diese Bewegung wird, wie bereits beschrieben, durch die kraftschlüssige Verbindung zwischen Stößel 14 und Haken 114 auf den Halter 54 übertragen, wodurch die Nadel 22 eingestochen wird. Nach dem Einstechen wird die kraftschlüssige Verbindung zwischen dem Stößel 14 und dem Haken 114 unterbrochen, und der Stößel 14 verschiebt den Kolben 23 um die eingestellte Distanz, also die Distanz zwischen zwei aufeinanderfolgenden Zähnen bzw. Zahnlücken 28, und bewirkt dadurch eine Injektion der eingestellten Flüssigkeitsmenge.

Es ist darauf hinzuweisen, daß der Haken 114 und die Spitze 124 auch durch entsprechende (nicht dargestellte) Teile im Inneren des Gehäuses 15 in bestimmten axialen Stellungen an einer Bewegung radial nach außen gehindert werden können, wie das anhand der Fig. 1 bis 6 in großer Ausführlichkeit erläutert wurde und für den Fachmann sofort verständlich ist. Jedoch genügt in vielen Fällen auch die dargestellte und beschriebene Ausführungsform gemäß Fig. 11A für einen zuverlässigen Betrieb, sofern die Vorspannkräfte der Fortsätze 112, 122 und die Winkel der Zähne 28 richtig gewählt sind. Versuche haben gezeigt, daß die Ausführungsform nach Fig. 11A durchaus zufriedenstellend und zuverlässig arbeitet. In vielen Fällen wird man jedoch aus Sicherheitsgründen im Gehäuse Kulissen vorsehen, welche analog zum Ausführungsbeispiel nach den Fig. 1 bis 6 die radialen Bewegungen der Fortsätze 112 und 122 in bestimmten Stellungen limitieren und dadurch Fehlfunktionen mit absoluter Sicherheit vermeiden.

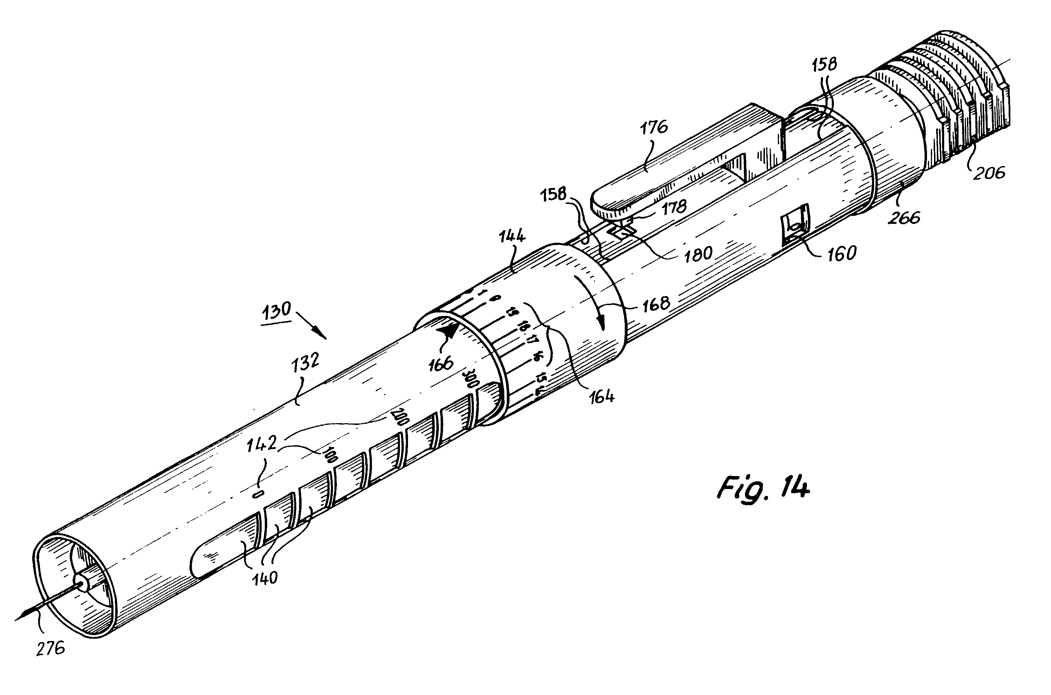

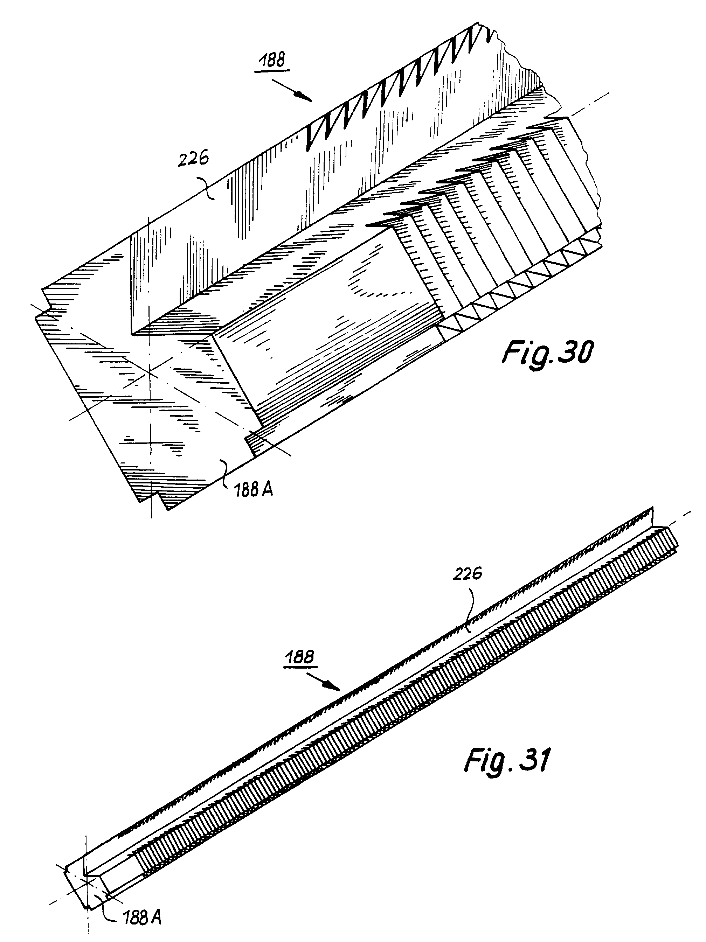

Die Fig. 14 bis 31 zeigen ein drittes Ausführungsbeispiel der Erfindung, bei dem die Injektionsdosis vom Benutzer in einfacher Weise verstellt werden kann, und bei dem besondere Maßnahmen getroffen sind, um eine exakte Einstellung auch kleiner Injektionsdosen zu ermöglichen. Kleine Dosen, z.B. bei Insulin 1 Einheit, erfordern nämlich bei den handelsüblichen Kartuschen sehr kleine Verstellwege, z.B. bei einem bekannten Produkt einen Weg des Kolbens in dieser Kartusche von nur 0,27 mm. Die Erfindung ermöglicht es, auch solche kleinen Dosiermengen präzise einzustellen und zu injizieren.

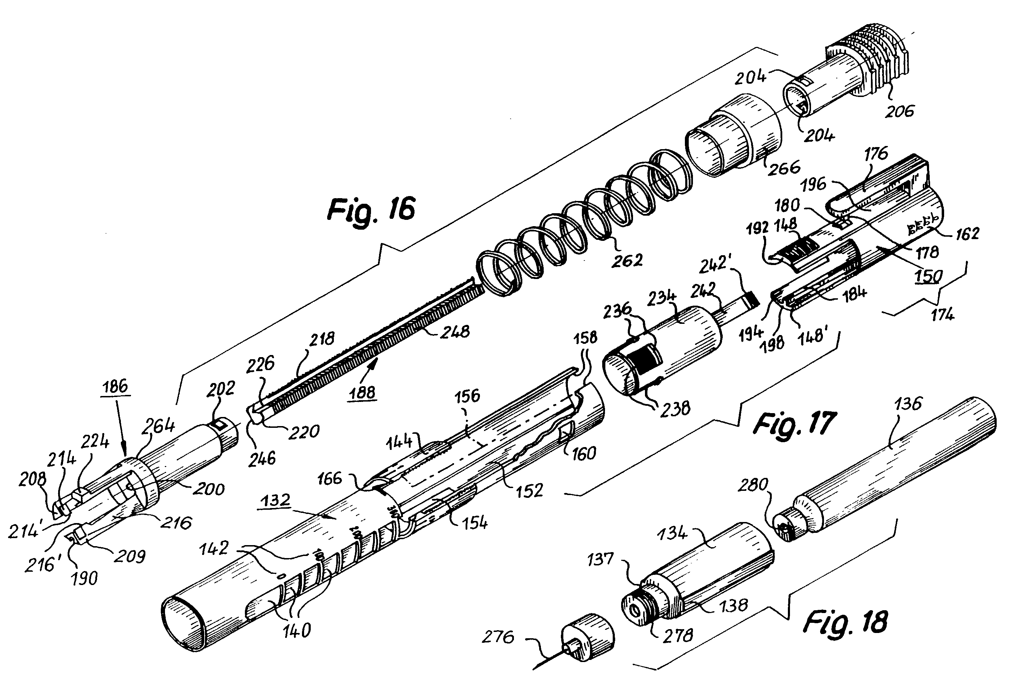

Bei diesem Ausführungsbeispiel weist das Gehäuse des Injektionsgeräts 130 mehrere Teile auf, und als Hauptteil ein rohrförmiges Gehäuseteil 132, dessen Gestalt am besten aus Fig. 17 hervorgeht, wobei in Fig. 17 rechts dieses Gehäuseteil 132 partiell weggeschnitten dargestellt ist, um einen Blick in sein Inneres zu gestatten.

Das Gehäuseteil 132 dient dazu, im Inneren seines proximalen Abschnitts einen durchsichtigen Halter 134 (Fig. 18) für eine ebenfalls durchsichtige Kartusche 136 üblicher Bauart zu führen. Dazu ist das Gehäuseteil 132 innen mit (nicht dargestellten) Führungsstegen versehen, welche längliche Vorsprünge 137, 138 des Halters 134 in Längsrichtung führen, so daß sich dieser Halter im proximalen Abschnitt des rohrförmigen Gehäuseteils 132 nicht verdrehen kann. Dieser proximale Abschnitt ist auch auf beiden Seiten mit Fenstern 140 versehen, durch welche man nachschauen kann, wieviel Flüssigkeit sich noch in der Kartusche 136 befindet. Zahlen 142 an diesen Fenstern gestatten eine grobe Schätzung des restlichen Inhalts der Kartusche 136. Die Fig. 14 und 17 zeigen nur die Fenster 140 auf einer Seite des rohrförmigen Gehäuseteils 132; diesen liegen symmetrisch auf der Gegenseite entsprechende Fenster gegenüber.

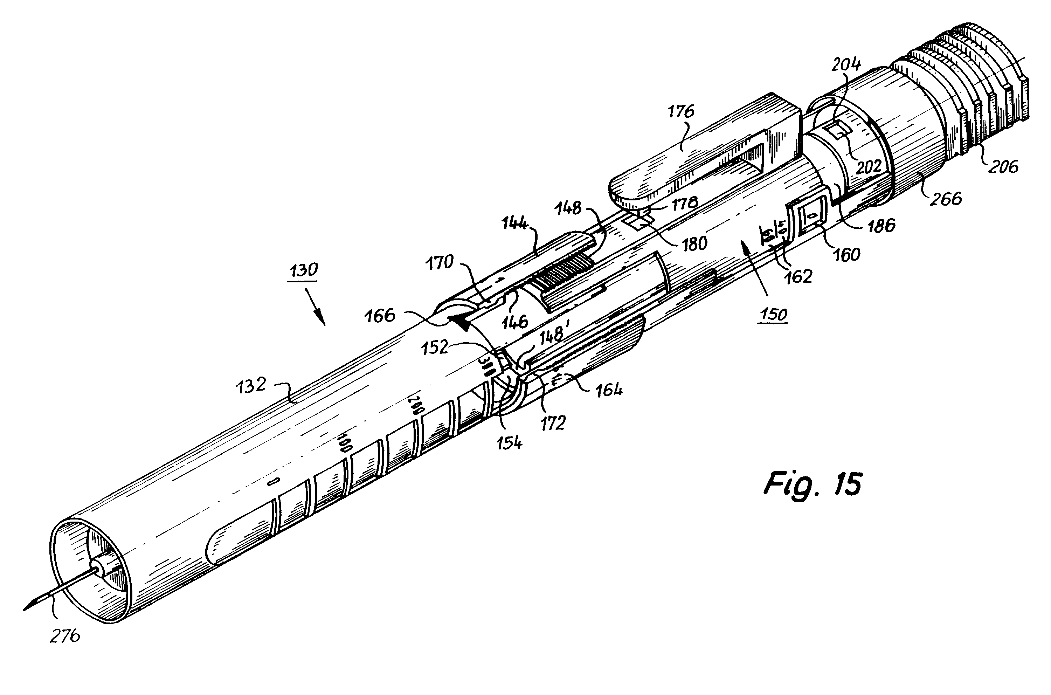

Etwa in der Mitte des rohrförmigen Gehäuseteils 132 ist auf diesem eine Gewindehülse 144 drehbar, aber axial unverschieblich, befestigt. Wie Fig. 15 am besten zeigt, hat die Hülse 144 auf ihrer Innenseite ein Innengewinde 146 in Gestalt eines Feingewindes, und dieses steht in Eingriff mit einem entsprechenden Außengewinde 148 eines zwecks Dosiseinstellung im Gehäuseteil 132 längsverschiebbaren Gehäuseteils 150, dessen Form am besten aus Fig. 17 hervorgeht.

Gemäß Fig. 17 hat das rohrförmige Gehäuseteil 132 in seinem distalen Bereich unten (bezogen auf Fig. 17) eine relativ breite Längsnut 152, die etwa in der Mitte des Gehäuseteils 132 ein rechteckförmiges Fenster 154 aufweist, dessen Form aus Fig. 17 klar ersichtlich ist. Durch dieses Fenster 154 ragt das Außengewinde 148' (Fig. 17) des Gehäuseteils 150 - durch die Wand des rohrförmigen Gehäuseteils 132 - radial nach außen, damit es mit dem Innengewinde 146 in Eingriff kommt.

Wie ferner in Fig. 17 - teilweise nur mit strichpunktierten Linien 156 - angedeutet, hat das rohrförmige Gehäuseteil 132 auf seiner distalen Seite einen Längsschlitz 158, der sich vom distalen Ende dieses Gehäuseteils bis fast zu dessen Mitte erstreckt. Der Längsschlitz 158 liegt dem Fenster 154 diametral gegenüber und ist gleich breit wie dieses. Durch den Längsschlitz 158 ragt das Außengewinde 148 des Gehäuseteils 150 radial nach außen, zwecks Eingriff mit dem Innengewinde 146 der Gewindehülse 144.

Das rohrförmige Gehäuseteil 132 hat ferner ein Sichtfenster 160, durch welches eine (grobe) Dosisanzeige 162 auf dem Gehäuseteil 150 abgelesen werden kann, vgl. Fig. 15. Die Feinanzeige der Dosis, z.B. von "0" bis "19", befindet sich in Form von Indicia 164 auf der Gewindehülse 144, und auf dem rohrförmigen Gehäuseteil 132 befindet sich ein zugeordneter Anzeigepfeil 166. Der Benutzer addiert also den Wert 162 im Fenster 160, z.B. "0", zum Wert 164, auf den der Pfeil 166 zeigt, um die augenblickliche Einstellung des Injektionsgeräts 130 zu erhalten, die in Fig. 4 Null beträgt. Dreht er dann die Hülse 144 in Richtung des Pfeiles 168 (Fig. 14) um drei Teilstriche weiter, so hat er eine Dosis von drei Einheiten eingestellt; diese Einstellung muß er nur dann verändern, wenn er anschließend eine andere Dosis injizieren will.

Beträgt die gewünschte Dosis beispielsweise stets 3 Einheiten, so kann die Gewindehülse 144 in dieser Stellung verbleiben. Das ist insbesondere für Diabetiker mit Sehschwäche von Vorteil, da diese dann vor einer Injektion keine Änderung der Einstellung der Gewindehülse 144 vornehmen müssen. Vielmehr muß ein solcher Patient dann lediglich das Gerät spannen, auf die gewünschte Körperstelle aufsetzen, und dann die Injektion auslösen, ohne sich um die Dosis zu kümmern.

Soll die Dosis geändert werden, so muß die Gewindehülse 144 entsprechend verdreht werden, wobei eine Drehung in Richtung des Pfeiles 164 eine Erhöhung der Injektionsdosis und eine Drehung entgegen dem Pfeil 164 eine Reduzierung der Injektionsdosis bewirkt.

Die Gewindehülse 144 ist, wie in Fig. 15 dargestellt, auf ihrer Innenseite mit einem ringförmigen Vorsprung (Bund) 170 versehen, der in eine zu ihm komplementäre Ringnut 172 auf der Außenseite des rohrförmigen Gehäuseteils 132 eingreift. Wie dargestellt, haben der Bund 170 und die Ringnut 172 schräge Flanken, so daß die Gewindehülse 144 einfach auf das Gehäuseteil 132 aufgepreßt werden kann, bis sie mit dem Bund 170 in die Ringnut 172 einrastet. Dadurch kann die Gewindehülse 144 nach ihrer Montage auf dem Gehäuseteil 132 zwar auf diesem verdreht, aber nicht axial relativ zu ihm verschoben werden.

Das Innengewinde 146 der Gewindehülse 144 ist so lang, daß es das Fenster 154 (Fig. 17) überdeckt.

Das Gehäuseteil 150 ist im rohrförmigen Gehäuseteil 132 linear verschiebbar aber nicht verdrehbar; beide Teile bilden zusammen das Gehäuse des Injektionsgeräts. Der distale Abschnitt 174 (Fig. 17) des Gehäuseteils 150 hat im wesentlichen die Form eines Rohres mit kreiszylindrischem Querschnitt, an dessen distalem Ende ein Clip 176 befestigt ist, der mit einem Vorsprung 178 zum Eingriff in eine radiale Rastöffnung 180 des Gehäuseteils 150 dient. Wird das Gehäuseteil 150 linear relativ zum Gehäuseteil 132 verschoben (durch Verdrehung der Gewindehülse 144), so verschiebt sich die Lage der Rastöffnung 180 relativ zum Gehäuseteil 132, und dies ermöglicht eine Verstellung der Injektionsdosis: Je mehr die Rastöffnung 180 in distaler Richtung verschoben wird, umso größer wird die Injektionsdosis.

Wie Fig. 17 und 23 am besten zeigen, hat das Gehäuseteil 150 in seinem Inneren einen axial verlaufenden Führungsvorsprung 184, der zur Führung eines Teils 186 (Fig. 16) dient, das im folgenden als Zustellteil bezeichnet wird, weil es bei einer Zahnstange 188 (Fig. 16, 30, 31), die in diesem Zustellteil geführt ist, eine Zustellbewegung in proximaler Richtung bewirkt. Hierzu hat das Zustellteil 186 eine in axialer Richtung verlaufende Nut 190 (Fig. 16), in welche der Führungsvorsprung 184 eingreift.

Auf seiner proximalen Seite hat das Gehäuseteil 150 zwei einander diametral gegenüberliegende, axiale Vorsprünge 192, 194, die bei der Montage in Richtung zueinander federnd auslenkbar sind. Der in Fig. 17 obere Vorsprung 192 trägt den Gewindeabschnitt 148, und der untere Abschnitt 194 den Gewindeabschnitt 148'. Auf dem oberen Vorsprung 192 verläuft eine längliche Verbreiterung 196, die nach der Montage vom Längsschlitz 158 des Gehäuseteils 132 geführt wird, und auf dem unteren Vorsprung 194 verläuft eine längliche Verbreiterung 198, die nach der Montage in der Nut 152 des Gehäuseteils 132 geführt ist. Dadurch ist das Gehäuseteil 150 im montierten Zustand, wie er in den Fig. 14 und 15 dargestellt ist, in Längsrichtung im Gehäuseteil 132 geführt und mit diesem dadurch verbunden, daß das Innengewinde 146 der Gewindehülse 144 in die Außengewindeabschnitte 148 und 148' des Gehäuseteils 150 eingreift.

Wie Fig. 16 zeigt, hat das Zustellteil 186 im Bereich seiner Längsachse eine Ausnehmung 200 mit quadratischem Querschnitt, in welcher die Zahnstange 188 längsverschiebbar geführt ist. Diese hat ebenfalls einen im wesentlichen quadratischen Querschnitt, wie sich das aus den Fig. 22, 30 und 31 ergibt. Das Zustellteil 186 hat auf seiner distalen Seite oben und unten einen Rastvorsprung 202, welche Rastvorsprünge bei der Montage in entsprechende Rastausnehmungen 204 eines Betätigungsknopfes 206 einrasten. (Das Zustellteil 186 ist in Fig. 15 teilweise weggeschnitten dargestellt, d.h. man sieht nur dessen distales Ende.)

Das Zustellteil 186 hat drei Führungsteile 208, 209, 210, von denen das Führungsteil 209 mit der Längsnut 190 versehen ist, vgl. Fig. 22. (In Fig. 16 ist das Führungsteil 210 - zwecks besserer Darstellung - nicht gezeigt.) Diese Führungsteile gleiten längs der Innenwand 212 des rohrförmigen Gehäuseteils 132 und führen dadurch das Zustellteil 186 in diesem.

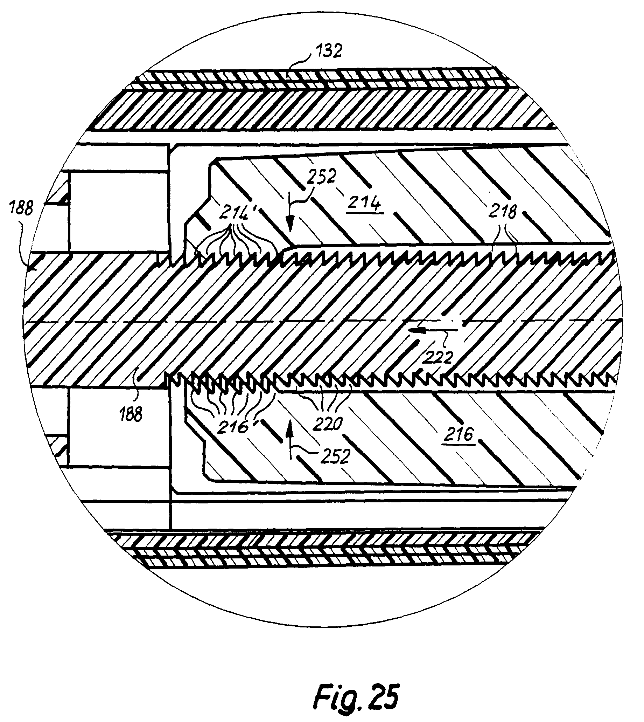

Ferner enthält das Zustellteil 186 zwei zangenartig ausgebildete, elastische Eingriffsglieder 214, 216, die an ihren freien Enden jeweils mit sieben Rastzähnen 214', 216' versehen sind. Diese Rastzähne sind sehr klein und deshalb nur in der Vergrößerung der Fig. 25 gut darstellbar.

Die Rastzähne 214', 216' liegen einander ohne axiale Versetzung direkt gegenüber, wie das Fig. 25 zeigt, während die ihnen entsprechenden Zahnungen 218 bzw. 220 auf den beiden hier wirksamen, gegenüberliegenden Seiten der Zahnstange 188 um eine halbe Zahnteilung gegeneinander versetzt sind. Deshalb greifen bei dieser Darstellung die Rastzähne 214' voll und mit Vorspannung in die Zähne 218 der Zahnstange 188 ein, während die Rastzähne 216' nur halb, und ebenfalls mit Vorspannung, im Eingriff mit den Zähnen 220 der Zahnstange 188 sind. (Naturgemäß können alternativ die Zähne auf der Zahnstange 188 einander ohne axiale Versetzung gegenüberliegen, und man würde dann die axiale Versetzung bei den Rastzähnen 214', 216' vorsehen, doch wird die dargestellte Form bevorzugt.)

Infolge der Form der Zähne kann sich die Zahnstange 188 ersichtlich nur in Richtung des Pfeiles 222 verschieben, also in proximaler Richtung, wobei die Eingriffsglieder 214, 216 elastisch radial nach außen ausgelenkt werden. Wird die Zahnstange 188 um eine halbe Zahnteilung in der Richtung 222 verschoben, so greifen die Zähne 216' voll in die Zähne 220 der Zahnstange 188 ein, und die Zähne 214' sind dann nur halb im Eingriff mit den Zähnen 218. Auf diese Weise kann ein Vorschub um eine halbe Zahnteilung realisiert werden, und eine halbe Zahnteilung entspricht hier beispielsweise 0,27 mm bzw. einer Einheit der zu injizierenden Flüssigkeit.

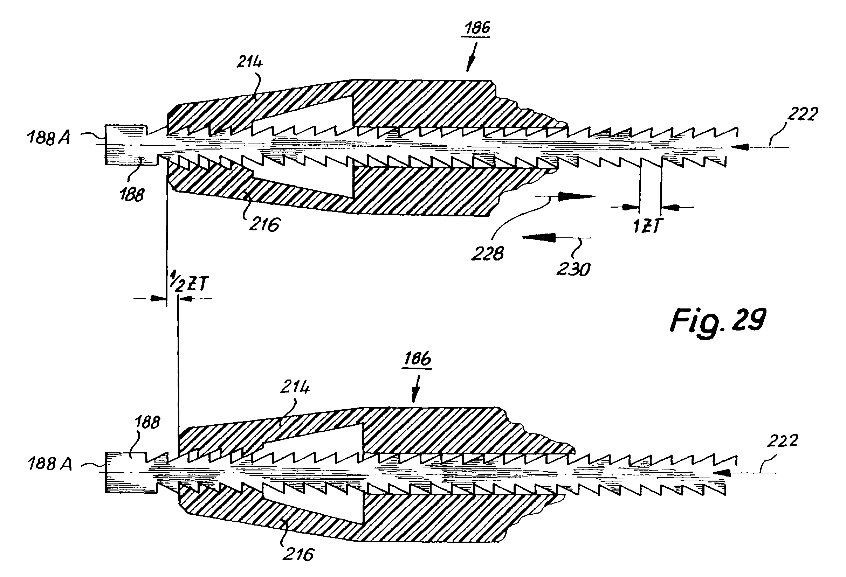

Fig. 29 zeigt in schematisierter Darstellung einen Vorschubvorgang der Zahnstange 222 um eine halbe Zahnteilung, was als "1/2 ZT" in der Zeichnung angegeben ist. Dies geschieht dadurch, daß das Zustellteil 186 zuerst in Richtung eines Pfeiles 228 in distaler Richtung um eine halbe Zahnteilung verschoben wird, wie im unteren Teil von Fig. 29 dargestellt, wobei die Zahnstange 188 in einer nachfolgend noch beschriebenen Weise festgehalten wird und die Zähne des Zustellteils 186 um eine halbe Zahnteilung weitergleiten, und daß das Zustellteil 186 anschließend in Richtung eines Pfeils 230 in proximaler Richtung verschoben wird, wodurch dann die Zahnstange 188 um eine halbe Zahnteilung in proximaler Richtung verschoben wird.

Am Zustellteil 186 ist ferner auch noch ein federndes Rastglied 224 vorgesehen, das beim Spannen des Injektionsgeräts 130 in die Rastöffnung 180 einrastet, und das durch Drücken auf den als Auslöser dienenden Clip 176 aus der Rastöffnung 180 herausgedrückt werden kann, um eine Injektion auszulösen, wie bei Fig. 8A und 9A ausführlich beschrieben. Wie aus Fig. 22 hervorgeht, liegt das Rastglied 224 im freien Raum zwischen zwei benachbarten Eingriffsgliedern 214, 244, und es liegt einer Kante der Zahnstange 188 gegenüber. Auf diese Weise gelingt es, das Injektionsgerät in raumsparender Weise aufzubauen, da für den Federweg des Rastglieds 224 auf diese Weise genügend Platz zur Verfügung steht.

Um die Zahnungen der Zahnstange 188 besser sichtbar zu machen, ist diese in Fig. 16, 30 und 31 mit einer Längsnut 226 dargestellt. Diese Längsnut 226 existiert in der Realität nicht, wie sich z.B. aus dem Schnitt der Fig. 22 klar ergibt. Sie dient lediglich zur besseren Veranschaulichung der Zahnungen.

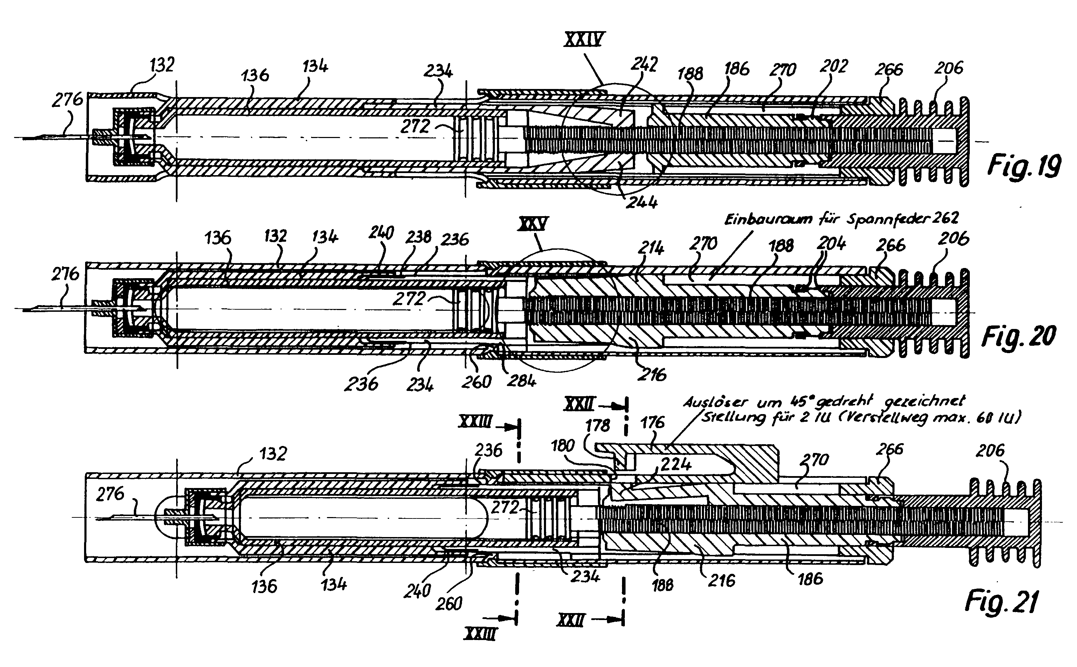

Das Injektionsgerät 130 enthält ferner ein Halterteil 234, das in Fig. 17 in raumbildlicher Form dargestellt ist und das im montierten Zustand durch einen Rastvorgang mit dem Halter 134 (Fig. 18) verbunden ist. Das Halterteil 234 hat auf seiner Außenseite Vorsprünge 236, deren Funktion nachfolgend erläutert wird, und es hat auf seiner Außenseite Mikrorastungen 238, die mit entsprechenden Mikrorastungen 240 (Fig. 20, 21) auf der Innenseite des distalen Endes des Halters 134 zusammenwirken.

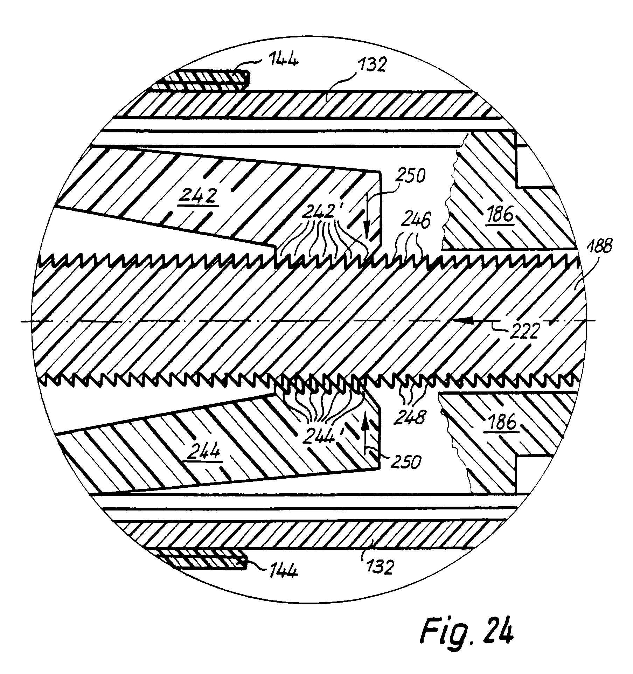

Das Halterteil 234 hat ferner, wie am besten aus Fig. 19 und 24 hervorgeht, an seinem distalen Ende zwei zangenartige, einander gegenüberliegende, elastisch auslenkbare Eingriffsglieder 242, 244. Die starke Vergrößerung gemäß Fig. 24 zeigt, daß diese Eingriffsglieder jeweils mit sieben Zähnen 242' bzw. 244' versehen sind, die einander ohne axiale Versetzungen gegenüberliegen, analog den Zähnen 214' und 216' der Fig. 25. (Im Prinzip genügt naturgemäß ein einziger Zahn, oder auch nur ein halber Zahn, doch wird eine größere Zahl von Zähnen bevorzugt.)

Die Zähne 242' und 244' greifen ein in entsprechende Zähne 246 bzw. 248 auf zwei gegenüberliegenden Seiten der Zahnstange 188. Diese Zähne 246, 248 sind relativ zueinander um eine halbe Zahnteilung versetzt, so daß immer nur entweder die Zähne 242' voll in die Zähne 246 eingreifen, wie in Fig. 24 dargestellt, oder umgekehrt nur die Zähne 244' voll in die Zähne 248 eingreifen.

Auch die Zähne 242', 244', 246 und 248 sind so ausgebildet, daß sie nur eine Verschiebung der Zahnstange 188 in proximaler Richtung (Pfeil 222) gestatten.

Die Eingriffsglieder 242, 244 sind so ausgebildet, daß sie mit Vorspannung gegen die Zahnstange 188 anliegen, wie in Fig. 24 durch Pfeile 250 angedeutet. Sie sind deshalb nach Art einer Spannzange ausgebildet.

Ebenso sind die Eingriffsglieder 214, 216 des Zustellteils 186 so ausgebildet, daß sie mit Vorspannung gegen die Zahnstange 188 anliegen, wie in Fig. 25 durch Pfeile 252 angedeutet.

Die Vorspannungen 250, 252 erhöhen die Reibung zwischen den betreffenden Eingriffsgliedern und der Zahnstange 188, was die Funktion verbessert und eine große Funktionssicherheit gewährleistet.

Der Umstand, daß die Vorspannungen 250, 252 jeweils symmetrisch auf die Zahnstange 188 wirken, verhindert, daß diese durch diese Kräfte verbogen wird.

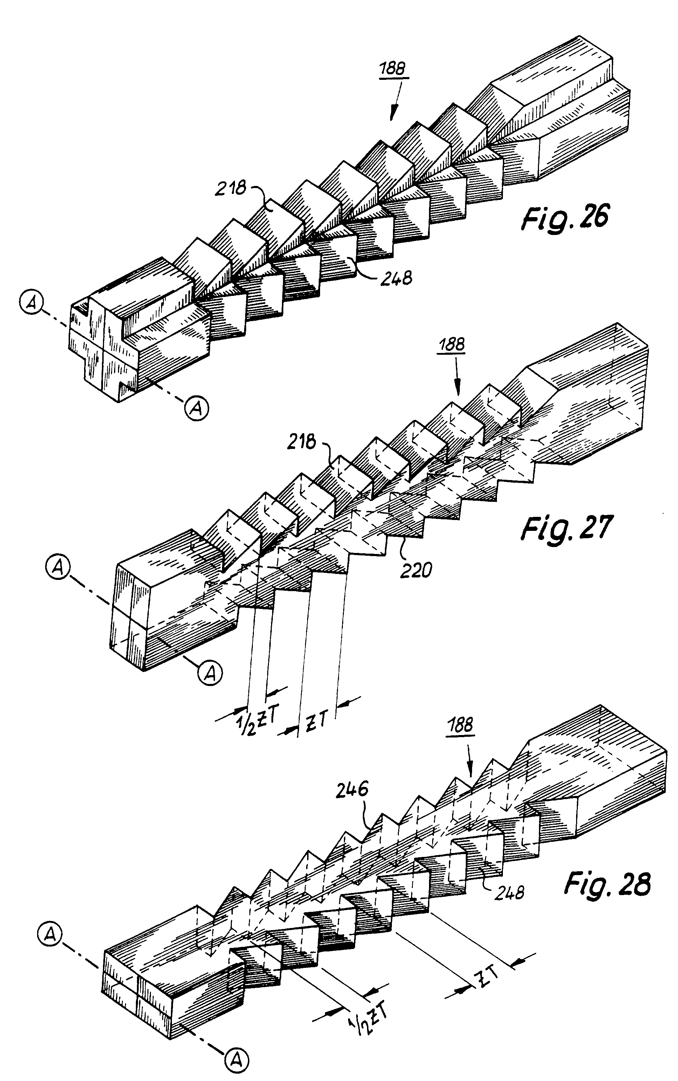

Das Verständnis der Struktur der Zahnstange 188 wird durch die Schemadarstellungen der Fig. 26 bis 28 erleichtert. In allen drei Figuren ist die Achse A-A dargestellt, um eine einfache Orientierung zu ermöglichen.

Fig. 26 zeigt die Zahnstange 188 raumbildlich und stark schematisiert, um das Verständnis zu erleichtern. Von den vier Zahnungen sieht man in Fig. 26 nur die beiden Zahnreihen 218 und 248, welche dieselbe Zahnteilung und dieselbe Phasenlage haben, d.h. ihre Täler liegen in derselben Ebene.

Fig. 27 zeigt dann nur die beiden gegenüberliegenden Zahnreihen 218 und 220, die mit dem Zustellteil 186 zusammenwirken und die um eine halbe Zahnteilung (1/2 ZT) gegeneinander versetzt sind, was man auch als Phasenverschiebung von 180° bezeichnen kann.

Fig. 28 zeigt nur die beiden gegenüberliegenden Zahnreihen 246 und 248, die ebenfalls um eine halbe Zahnteilung (1/2 ZT) gegeneinander versetzt sind, also ebenfalls eine Phasenverschiebung von 180° haben.

Die Zahnreihen 246 und 248 wirken mit dem Halterteil 234 (Fig. 17) zusammen und dienen zu dessen Antrieb, analog dem Antrieb des Teils 66 in Fig. 11A.

### Montage

Zunächst wird auf das rohrförmige Gehäuseteil 132 die Gewindehülse 144 in der bereits beschriebenen Weise aufgerastet. Dann wird das Teil 234 in das Gehäuseteil 132 von dessen distaler Seite aus eingeschoben. Das Teil 234 gleitet mit seinen Rastvorsprüngen 236 über einen entsprechenden inneren Rastwulst 260 des Gehäuseteils 132. Die Rastvorsprünge 236 sind so ausgebildet, daß sie eine solche Verschiebung in proximaler Richtung ermöglichen, nicht aber in distaler Richtung, da sie dann als Anschläge wirken, wie in Fig. 21 dargestellt. (In Fig. 21 ist der als Auslöser dienende Clip 176 um 45° verdreht gezeichnet, d.h. er wäre in dieser Schnittdarstellung eigentlich nicht sichtbar. Fig. 21 zeigt die gespannte Stellung für die Injektion von zwei Einheiten, entsprechend einer einzigen Zahnteilung der Zahnstange 188.)

Anschließend wird das Gehäuseteil 150 (Fig. 17) in das rohrförmige Gehäuseteil 132 eingeschoben, wobei das Gewinde 148' in das Fenster 154 im Gehäuseteil 132 einrastet, und durch Drehen der Gewindehülse 144 wird das Gehäuseteil 150 in seine Nullstellung gebracht, die im Fenster 160 angezeigt wird. Damit ist das Gehäuse fertig aus den Teilen 132, 144 und 150 zusammengesetzt und hat die Form, die aus Fig. 14 hervorgeht. Dies entspricht der Nullstellung, also der Stellung für die Injektion von 0 Einheiten.

Nun führt man von der distalen Seite dieses Gehäuses das Zustellteil 186 in das Gehäuse ein. In die Öffnung 200 des Zustellteils 186 steckt man die Zahnstange 188 so weit ein, daß die Eingriffsglieder 214, 216 des Zustellteils 186 in die ersten Zähne am proximalen Ende der Zahnstange 188 einrasten. Dies entspricht etwa der Stellung gemäß Fig. 25, aber noch um einen Zahn weiter nach links verschoben.

Anschließend setzt man eine Spannfeder 262 (Fig. 16) vom distalen Ende in das rohrförmige Gehäuseteil 132 ein, und zwar bis zum Anschlag gegen den Bund 264 des Zustellteils 186, wobei das Zustellteil 186 zweckmäßig mit seinem federnd auslenkbaren Rastvorsprung 224 in der Rastöffnung 180 verrastet wird. Dann wird ein Bundteil 266 durch Rasten oder Kleben im distalen Ende des rohrförmigen Gehäuseteils 132 befestigt. Es dient als Widerlager für die Spannfeder 262.

In den Fig. 19 bis 21 ist nur der Ringraum 270 dargestellt, in welchem sich die Spannfeder 262 nach dem Einbau befindet, da diese Zeichnungen sehr unübersichtlich geworden wären, wenn auch die Spannfeder 262 dort dargestellt würde. Bis auf die Spannfeder 262 können alle Teile des Injektionsgeräts 130 aus einem geeigneten Kunststoff hergestellt werden.

Nach der Befestigung des Bundteils 266 wird der Betätigungsknopf 206 am distalen Ende des Zustellteils 186 befestigt, wie bereits beschrieben (Teile 202, 204 in Fig. 16).

Die Mechanik ist nun weitgehend fertig montiert, und das proximale Ende 188A (Fig. 29) der Zahnstange 188 steht zur Betätigung des Kolbens 272 (Fig. 19 bis 21) der Kartusche 136 bereit. Die Eingriffsglieder 214, 216 des Zustellteils 186 und die Eingriffsglieder 242, 244 des Halterteils 234 greifen dabei interdigital ineinander, wie sich das aus der Schnittdarstellung der Fig. 22 klar ergibt. Dies ist sehr ähnlich Fig. 11A, läßt sich aber in einem Längsschnitt nicht darstellen. Ein Vergleich der Fig. 19 und 20 zeigt dieses Ineinandergreifen.

Man löst nun durch Druck auf den Auslöser 176 die Verrastung mit dem Rastglied 224 aus, wodurch - durch die Wirkung der Feder 262 - die Zahnstange 188 die Lage gemäß Fig. 19 und 20 einnimmt, und führt dann vom proximalen Ende her die Kartusche 136 in das rohrförmige Gehäuseteil 132 ein, bis ihr Kolben 272 gegen das proximale Ende 188A der Zahnstange 188 anliegt. Anschließend führt man, ebenfalls vom proximalen Ende des Gehäuseteils 132 her, den Halter 134 in dieses Gehäuseteil ein. Durch eine entsprechende Kraft wird dessen innere Mikrorastung 240 zur Verrastung gebracht mit der Mikrorastung 238 auf dem Halterteil 234. Hierbei wird die Kraft gemessen, die für diese Verrastung notwendig ist. Wenn diese Kraft ansteigt, weil der Kolben 272 der Kartusche 136 zur Anlage gegen das proximale Ende 188A der Zahnstange 188 kommt, wird die Verrastung beendet, da sich dann der Halter 134 in der richtigen Stellung auf dem Halterteil 234 befindet.

Das Gerät ist nun fertig, und durch Aufschrauben einer Nadel 276 auf ein Gewinde 278 am proximalen Ende des Halters 134 kann es vom Patienten benutzungsbereit gemacht werden. Diese Nadel 276 durchdringt dabei mit ihrem distalen Ende eine Gummimembran 280 (Fig. 18) am proximalen Ende der Kartusche 136. (Die Nadel 276 sollte nach jeder Injektion gewechselt werden. Sie wird erst vor einer Injektion befestigt und wird bis dahin in einem sterilen Behälter aufbewahrt.)

### Arbeitsweise

Vor einer Injektion stellt man durch Drehen an der Gewindehülse 144 die gewünschte Dosis ein, z.B. 2 Einheiten, wie in Fig. 21 dargestellt. Dadurch wird das Gehäuseteil 150 mit seiner Rastöffnung 180 entsprechend weit in distaler Richtung relativ zum Gehäuseteil 132 verschoben, z.B. bei 2 Einheiten um eine Zahnteilung der Zahnstange 188, entsprechend beispielsweise 0,54 mm. Deshalb ist dies in Fig. 21 zeichnerisch nicht darstellbar.

Anschließend wird durch Ziehen in distaler Richtung am Betätigungsknopf 206 die Spannfeder 262 (Fig. 16) gespannt, und der Rastvorsprung 224 wird zum Einrasten in die Rastöffnung 180 gebracht.

Bei dieser Spannbewegung wird durch die Eingriffsglieder 242, 244 (Fig. 19), die wie Spannbacken wirken und den Halter 134 mitnehmen, die Nadel 276 ins rohrförmige Gehäuseteil 132 hineingezogen, vgl. Fig. 21. Dabei gelangen die Vorsprünge 236 des Halterteils 234 (am besten sichtbar in Fig. 17) zum Anschlag gegen den Bund 260 im Gehäuseteil 132, und wenn dies der Fall ist, gleiten die Zähne 214', 216' um die eingestellte eine Zahnteilung über die Zähne 218, 220 der Zahnstange 188 hinweg und stellen so die Dosis ein, wobei anschließend das Rastglied 224 gemäß Fig. 21 in die Ausnehmung 180 einrastet.

Nach dem Auslösen mittels des Auslösers 176 verschiebt die gespannte Feder 262 über die Eingriffsglieder 214, 216 die Zahnstange 188 in proximaler Richtung, und durch die wie Klemmbacken wirksamen Eingriffsglieder 242, 244 des Halterteils 234 wird diese Bewegung direkt auf die Nadel 276 übertragen, so daß diese in das Gewebe des Patienten einsticht. Dabei kommt das Halterteil 234 mit einem radialen Vorsprung 284 (Fig. 20) zum Anschlag gegen den Ringbund 260 im Inneren des Gehäuseteils 132 und begrenzt so die Einstichtiefe der Nadel 276.

Da aber die Zahnstange 188 ihre Bewegung in proximaler Richtung (Pfeil 222 in Fig. 24) noch fortsetzt, gleitet sie (bei diesem Beispiel, bei dem eine Zahnteilung als Injektionsdosis eingestellt wurde) in Fig. 24 relativ zu den Eingriffsgliedern 242, 244 in Richtung des Pfeiles 222 um eine Zahnteilung weiter, wodurch die entsprechende Flüssigkeitsmenge (2 Einheiten) durch Verschiebung des Kolbens 272 aus der Kartusche 136 ausgepreßt wird. Ersichtlich ist in gleicher Weise eine Verschiebung auch nur um eine halbe Zahnteilung, 1,5 Zahnteilungen, etc. möglich, und dies wird dadurch erreicht, daß die Anordnungen nach Fig. 24 und 25 im Prinzip gleich ausgebildet sind.

Auf diese Weise ist beim Ausführungsbeispiel eine Dosiseinstellung von 1 ... 60 Einheiten möglich, was den Bedarf der Praxis abdeckt.

Naturgemäß sind im Rahmen der vorliegenden Erfindung vielfache Abwandlungen und Modifikationen möglich. So könnte man z.B. statt der Versetzung der Zähne um eine halbe Zahnteilung, wie sie in den Fig. 24 bis 29 dargestellt ist, eine Anordnung mit drei Zahnreihen verwenden, von denen jede nur um 1/3 Zahnteilung gegen die andere versetzt ist, wobei jeder Zahnreihe dann ein entsprechendes Eingriffsglied zuzuordnen ist. Solche und andere Modifikationen ergeben sich für den Fachmann ohne Schwierig-keiten und liegen im Rahmen der Erfindung.

## Patentansprüche

1. Injektionsgerät, welches zur Abgabe mehrerer Injektionsdosen geeignet ist,
mit einem Gehäuse (15; 132, 150),
mit einem relativ zu diesem Gehäuse zwischen einer distalen Endstellung (Fig. 2, 3, 9, 21) und einer proximalen Endstellung (Fig. 1, 4, 5, 8, 11, 19, 20) verschiebbaren Behälter (12; 136) für eine Injektionsflüssigkeit, oder einem Halter (134, 234) für einen solchen Behälter,
mit einem relativ zum Gehäuse beweglichen Stößel (14; 188) zum Auspressen von Flüssigkeit aus einem solchen Behälter (12; 136),
und mit einer zwischen dem Stößel (14; 188) und dem Behälter (12; 136). oder einem Halter (134, 234) für diesen Behälter, vorgesehenen ersten Verbindungsanordnung (27; 112, 114; 242, 244, 246, 248), welche dazu ausgebildet ist, bei einer Bewegung des Stößels (14; 188) in distaler Richtung eine unidirektionale Zwangsverbindung zwischen Stößel (14; 188) und Behälter (12; 136); oder einem Halter (134, 234) für den Behälter, herzustellen und letztere(n) zusammen mit dem Stößel (14; 188) in distaler Richtung zu bewegen, wobei
diese erste Verbindungsanordnung dazu ausgebildet ist, eine Bewegung des Stößels (14; 188) in proximaler Richtung während einer vorgegebenen Wegstrecke (S) dieser Bewegung mittels einer während dieser Wegstrecke (S) wirksamen mechanischen Verbindung zwischen Stößel (14; 188) und Behälter (12; 136), oder zwischen Stößel und einem Halter (134, 234) für diesen Behälter, in eine entsprechende proximal gerichtete Bewegung des Behälters (12; 136), oder eines Halters (134, 234) für den Behälter, umzusetzen.

2. Injektionsgerät nach Anspruch 1, bei welchem die unidirektionale Zwangsverbindung so ausgebildet ist, dass ein Anschlag (18, 20; 70, 73; 260) für die distale Bewegung des Behälters (12; 136), oder eines Halters (134, 234) für den Behälter, auch eine Begrenzung für die distale Bewegung des Stößels (14; 188) bildet.

3. Injektionsgerät nach Anspruch 1 oder 2, bei welchem die erste Verbindungsanordnung (27; 112, 114; 242, 244, 246, 248) so ausgebildet ist, dass sie in der distalen Endstellung des Behälters (12; 136), oder eines Halters (134, 234) für den Behälter, sowohl in proximaler wie in distaler Richtung wirksam ist,
und dass sie in der proximalen Endstellung des Behälters (12; 136), oder eines Halters für den Behälter, nur bei Bewegung des Stößels (14; 188) in distaler Richtung wirksam ist.

4. Injektionsgerät nach einem oder mehreren der Ansprüche 1 bis 3, bei welchem ein Antriebsorgan (35; 186) für den Stößel (14; 188) vorgesehen ist,
und zwischen dem Antriebsorgan (35; 186) und dem Stößel (14; 188) eine weg- und/oder richtungsabhängig wirksame zweite Verbindungsanordnung (38; 122, 124; 214, 216, 218, 220) vorgesehen ist.

5. Injektionsgerät nach Anspruch 4, bei welchem die zweite Verbindungsanordnung (38; 122, 124; 214, 216, 218, 220) dazu ausgebildet ist, bei einer Bewegung des Antriebsorgans (35; 186) in proximaler Richtung eine unidirektionale Zwangsverbindung zwischen Antriebsorgan (35; 186) und Stößel (14; 188) herzustellen und letzteren zusammen mit dem Antriebsorgan (35; 186) in proximaler Richtung zu bewegen,
und eine Bewegung des Antriebsorgans (35; 186) in distaler Richtung so weit in eine entsprechende Bewegung des Stößels (14; 188) in distaler Richtung umzusetzen, wie der Stößel (14; 188) an einer Bewegung in distaler Richtung nicht gehindert ist.

6. Injektionsgerät nach Anspruch 4 oder 5, bei welchem die zweite Verbindungsanordnung (38) in einem proximalen Stellungsbereich (Fig. 1) des Behälters (12) eine Verbindung zwischen Antriebsorgan (35) und Stößel (14) sowohl in proximaler wie in distaler Richtung herstellt.

7. Injektionsgerät nach einem oder mehreren der Ansprüche 4 bis 6, bei welchem die zweite Verbindungsanordnung (38; 214, 216, 218, 220) in einem distalen Stellungsbereich (Fig. 2, 3, 21) des Behälters (12; 136) eine in proximaler Richtung wirksame Verbindung zwischen Antriebsorgan (35; 186) und Stößel (14; 188) herstellt, welche eine Verschiebung des Antriebsorgans (35; 186) relativ zum Stößel (14; 188) in distaler Richtung ermöglicht.

8. Injektionsgerät nach einem oder mehreren der Ansprüche 4 bis 7, bei welchem dem Antriebsorgan (35; 186) eine auf dieses in proximaler Richtung wirkende Feder (11; 262), insbesondere eine aus Kunststoffmaterial hergestellte Feder, und ein Rastorgan (46; 94; 224) zugeordnet sind, welche Feder (11; 262) durch Verschieben des Rastorgans (46; 94; 224) in distaler Richtung spannbar ist,
wobei das Rastorgan bei Erreichen einer vorgegebenen Spannstellung (Fig. 3, 9, 21) einrastet.

9. Injektionsgerät nach Anspruch 8, bei welchem ein an der Außenseite des Gehäuses (15; 150) vorgesehener Clip (98; 176) zum Auslösen des Rastorgans (94; 224) und damit eines Injektionsvorganges ausgebildet ist.

10. Injektionsgerät nach einem oder mehreren der Ansprüche 4 bis 9, bei welchem dem Antriebsorgan (35; 186) ein Betätigungsglied (80; 206) zugeordnet ist, welches bevorzugt im distalen Bereich des Geräts angeordnet ist und ein Verschieben des Antriebsorgans (35; 186) in dessen Spannstellung (Fig. 9, 21) ermöglicht.

11. Injektionsgerät nach einem oder mehreren der Ansprüche 4 bis 10, bei welchem die unidirektionale Zwangsverbindung zwischen Stößel (14; 188) und Behälter (12; 136) und/oder zwischen Antriebsorgan (35; 186) und Stößel (14; 188) nach Art einer einseitig wirkenden Antriebskupplung ausgebildet ist, insbesondere nach Art einer Ratschenkupplung.

12. Injektionsgerät nach Anspruch 11, bei welchem die Verbindungsanordnung in der zur unidirektionalen Zwangsverbindung entgegengesetzten Richtung als kraftschlüssige Verbindungsanordnung und/oder als wegabhängig gesteuerte formschlüssige Verbindungsanordnung ausgebildet ist.

13. Injektionsgerät nach einem oder mehreren der vorhergehenden Ansprüche, bei welchem die erste Verbindungsanordnung ein mit dem Behälter (12; 136) verbundenes, zum Eingriff mit dem Stößel (14; 188) ausgebildetes Glied (27; 112; 242, 244) aufweist.

14. Injektionsgerät nach Anspruch 13, bei welchem das mit dem Behälter (12; 136) verbundene Glied (17; 112; 242, 244) zumindest in der proximalen Endstellung des Behälters (12; 136) in federndem Eingriff mit dem Stößel (14; 188) steht.

15. Injektionsgerät nach Anspruch 13 oder 14, bei welchem dem Behälter (12; 136) ein Halter (17; 54; 134, 234) zugeordnet ist, welcher relativ zum Gehäuse (15; 132, 150) verschiebbar ausgebildet ist,
wobei das zum Eingriff mit dem Stößel (14; 188) ausgebildete Glied (27; 112; 242, 244) an diesem Halter (17; 54; 134, 234) angeordnet und mit diesem verschiebbar ist.

16. Injektionsgerät nach einem oder mehreren der vorhergehenden Ansprüche, bei welchem die zweite Verbindungsanordnung ein am Antriebsorgan (35; 186) vorgesehenes, zum Eingriff mit dem Stößel (14; 188) ausgebildetes Glied (38; 112; 186) aufweist, welches zumindest im distalen Endstellungsbereich des Behälters (12; 136) in federndem Eingriff mit dem Stößel (14; 188) steht.

17. Injektionsgerät nach einem oder mehreren der Ansprüche 13 bis 16, bei welchem das zum Eingriff mit dem Stößel (14) ausgebildete Glied (27; 38) je nach der Stellung des Behälters (12), oder eines Halters (134, 234) für diesen Behälter, durch ein gehäusefestes Glied oder dergleichen an einer federnden Bewegung weg vom Stößel (14) gehindert ist.

18. Injektionsgerät nach einem oder mehreren der vorhergehenden Ansprüche, bei welchem der Stößel (14; 188) mindestens bereichsweise nach Art einer Zahnstange ausgebildet ist (Fig. 10; 30, 31).

19. Injektionsgerät nach Anspruch 18, bei welchem die Zähne (28; 218, 248) der Zahnstange (14; 188) so ausgebildet sind, dass sie relativ zu einem federnden Rastorgan nur eine Bewegung der Zahnstange in proximaler Richtung gestatten.

20. Injektionsgerät nach einem oder mehreren der vorhergehenden Ansprüche, bei welchem dem Stößel (14; 188) ein Anschlag (91) zugeordnet ist, welcher zumindest dessen proximale Bewegung relativ zum Gehäuse (15; 132) begrenzt.

21. Injektionsgerät nach einem oder mehreren der vorhergehenden Ansprüche, bei welchem dem Behälter (12; 136) ein Halter (17; 54; 134, 234) zugeordnet ist, welcher im Gehäuse (15; 132) axial zwischen einer proximalen und einer distalen Endstellung verschiebbar und zur Aufnahme eines Behälters (12; 136) ausgebildet ist.

22. Injektionsgerät nach Anspruch 21, bei welchem die Länge des Halters (54; 134, 234) veränderbar ist.

23. Injektionsgerät nach Anspruch 22, bei welchem der Halter (54; 134, 234) einen proximalen Abschnitt (56; 134) und einen distalen Abschnitt (66; 234) aufweist, welche durch eine verstellbare Verbindung (64; 236, 240) miteinander verbunden sind, die z.B. mittels einer axialen Kraft (Fig. 8A: K) eine Änderung und insbesondere eine Verkürzung der Gesamtlänge des Halters (54; 134, 234) ermöglicht, insbesondere durch eine Mikrorastung.

24. Injektionsgerät nach einem oder mehreren der Ansprüche 21 bis 23, bei welchem der Halter (54; 134, 234) in seiner proximalen Endstellung mit seinem distalen Ende gegen einen proximalen Endabschnitt des Antriebsglieds anliegt.

25. Injektionsgerät nach einem oder mehreren der vorhergehenden Ansprüche, bei welchem die Wirksamkeit der ersten und/oder der zweiten Verbindungsanordnung (27; 38; 112, 114; 122, 124; 242, 244, 246, 248, 214, 216, 218, 220) eine Funktion der jeweiligen Lage des Behälters (12; 136) ist.

26. Injektionsgerät nach einem oder mehreren der Ansprüche 21 bis 24, bei welchem der Halter (54; 134, 234) in seinem distalen Bereich mit Anschlagmitteln (68, 73; 236) versehen ist, welche seine proximale und/oder seine distale Endstellung relativ zum Gehäuse (15; 132) festlegen,
und bei welchem die Längenverstellung des Halters (54; 134, 234) relativ zu diesen Anschlagmitteln erfolgt, so dass bei einer Längenänderung des Halters (54; 134, 234) die Lage des Behälters (12; 136) relativ zum Stößel (14; 188) verändert wird.

27. Injektionsgerät nach Anspruch 26, bei welchem die Einrichtung zur Längenverstellung eine Mikrorastung (64; 236, 240) aufweist, welche einen proximalen Abschnitt (56; 134) und einen distalen Abschnitt (66; 234) des Halters (54) in verstellbarer Weise miteinander verbindet.

28. Injektionsgerät nach einem oder mehreren der vorhergehenden Ansprüche, bei welchem das Gehäuse mindestens zweiteilig ausgebildet ist und eine Vorrichtung (144) zur Veränderung der relativen Lage von Gehäuseteilen (132, 150) insbesondere durch lineare relative Verschiebung zwischen diesen, vorgesehen ist.

29. Injektionsgerät nach Anspruch 28, bei welchem ein erstes Gehäuseteil (132) zur Führung des den Behälter (136) für die Injektionsflüssigkeit aufnehmenden Halters (134, 234) vorgesehen ist und ein zweites, relativ zum ersten verschiebbares Gehäuseteil (150) Rastmittel (180) zur Verrastung eines am Antriebsorgan (186) vorgesehenen Rastorgans (224) aufweist.

30. Injektionsgerät nach Anspruch 29, bei welchem auf der Außenseite des zweiten Gehäuseteils (150) ein Clip (176) zum Lösen der Rastung zwischen den Rastmitteln (180) und dem Rastorgan (224) vorgesehen ist.

31. Injektionsgerät nach einem oder mehreren der Ansprüche 28 bis 30, bei welchem an einem der relativ zueinander verschiebbaren Gehäuseteile (150) ein Gewinde (148, 148'), und am anderen Gehäuseteil (132) eine relativ zu letzterem verdrehbare, aber relativ zu ihm nicht axial verschiebbare Gewindehülse (144) vorgesehen ist, welche mit diesem Gewinde (148, 148') in Eingriff steht.

32. Injektionsgerät nach einem oder mehreren der Ansprüche 28 bis 31, bei welchem Führungsmittel (152, 156, 196, 198) zur linearen Führung zwischen den relativ zueinander verschiebbaren Gehäuseteilen (132, 150) vorgesehen sind.

33. Injektionsgerät nach einem oder mehreren der Ansprüche 28 bis 32, bei welchem eines der Gehäuseteile (150) im anderen (132) geführt ist.

34. Injektionsgerät nach Anspruch 33, bei welchem das äußere Gehäuseteil (132) mit mindestens einer länglichen Ausnehmung (158) versehen ist, durch welche ein Abschnitt (196) des inneren Gehäuseteils (150) radial nach außen ragt.

35. Injektionsgerät nach Anspruch 34, bei welchem der nach außen ragende Teil (196) des inneren Gehäuseteils (150) mit einem Außengewinde (148, 148') versehen ist, welcher mit dem Innengewinde (146) einer auf dem äußeren Gehäuseteil (132) drehbar, aber axial nicht verschiebbar angeordneten Gewindehülse (144) in Eingriff steht.

36. Injektionsgerät nach Anspruch 31 oder 35, bei welchem die Gewinde (146, 148, 148') als Feingewinde ausgebildet sind.

37. Injektionsgerät nach einem oder mehreren der vorhergehenden Ansprüche, bei welchem die erste Verbindungsanordnung (186) zwei Eingriffsglieder (214, 216) aufweist, welche mit Eingriffselementen (214', 216') versehen sind, die zum Eingriff in entsprechende Zahnreihen (218, 220) einer als Stößel dienenden Zahnstange (188) ausgebildet sind.

38. Injektionsgerät nach einem oder mehreren der vorhergehenden Ansprüche, bei welchem die zweite Verbindungsanordnung zwei Eingriffsglieder (242, 244) aufweist, welche mit Eingriffselementen (242', 244') versehen sind, die zum Eingriff in entsprechende Zahnreihen (246, 248) einer als Stößel dienenden Zahnstange (188) ausgebildet sind.

39. Injektionsgerät nach den Ansprüchen 37 und 38, bei welchem die als Stößel dienende Zahnstange (188) einen rechteckförmigen und bevorzugt quadratischen Querschnitt aufweist und auf zwei ersten gegenüberliegenden Seiten mit Zahnreihen (218, 220) für die erste Verbindungsanordnung und auf den beiden anderen gegenüberliegenden Seiten mit Zahnreihen (246, 248) für die zweite Verbindungsanordnung versehen ist.

40. Injektionsgerät nach Anspruch 39, bei welchem die Eingriffsglieder (214, 216) der ersten Verbindungsanordnung und die Eingriffsglieder (242, 244) der zweiten Verbindungsanordnung interdigital ineinandergreifen.

41. Injektionsgerät, nach einem oder mehreren der Ansprüche 37 bis 40, bei welchem die Zahnreihen (218, 220) auf den einander gegenüberliegenden Seiten einer als Stößel dienenden Zahnstange (188) und/oder die Eingriffselemente (214', 216') auf mit diesen gegenüberliegenden Zahnreihen (218, 220) in federndem Eingriff stehenden Eingriffsgliedern (214, 216) gegeneinander in axialer Richtung versetzt sind, um alternativ einen vollen Eingriff eines Eingriffselements des ersten von zwei federnden Eingriffsgliedern mit der ihm zugeordneten Zahnreihe des Stößels, oder einen vollen Eingriff eines Eingriffselements des zweiten von zwei federnden Eingriffsgliedern mit der ihm zugeordneten Zahnreihe des Stößels (188), und damit eine verfeinerte Einstellmöglichkeit des Injektionsgeräts, zu ermöglichen.

42. Injektionsgerät nach einem oder mehreren der Ansprüche 37 bis 41, bei welchem die Zahnstange (188), im Querschnitt gesehen, als Polygon ausgebildet und auf einer Mehrzahl von Seiten dieses Polygons jeweils mit einer Zahnreihe (218, 220, 246, 248) versehen ist, welche Zahnreihen mit einem mit der Zahnstange (188) zusammenwirkenden, axial verschiebbaren Gegenglied, insbesondere einem Betätigungsglied, zusammenwirken, welches Gegenglied eine Mehrzahl von Eingriffsgliedern (214, 216) aufweist, die jeweils mit mindestens einem zur zugeordneten Zahnreihe komplementären Eingriffselement (214', 216') versehen sind und über dieses Eingriffselement in federndem Eingriff mit der zugeordneten Zahnreihe stehen,
wobei jeweils ein Eingriffsglied aus dieser Mehrzahl von Eingriffsgliedern (214, 216) über sein mindestens eines komplementäres Eingriffselement (214', 216') mit der zugeordneten Zahnreihe (218, 220) in vollem Eingriff steht,
und andere Eingriffsglieder aus dieser Mehrzahl von Eingriffsgliedern mit der ihnen jeweils zugeordneten Zahnreihe nicht in vollem Eingriff stehen.

43. Injektionsgerät nach Anspruch 42, bei welchem die mit dem Gegenglied zusammenwirkenden Zahnreihen (218, 220) der Zahnstange (188) im wesentlichen symmetrisch zur Längsachse der Zahnstange (188) angeordnet sind.

44. Injektionsgerät nach Anspruch 42 oder 43, bei welchem das Gegenglied als Antriebsorgan (186) zur Verschiebung des Behälters (136) im Gehäuse (132, 150) des Injektionsgeräts ausgebildet ist.

45. Injektionsgerät nach einem oder mehreren der Ansprüche 42 bis 44, bei welchem die Zahnstange (188) einen rechteckförmigen, insbesondere quadratischen, Querschnitt aufweist,
und das Gegenglied zwei Eingriffsglieder (214, 216) aufweist, die mit Zahnreihen (218, 220) auf einander gegenüberliegenden Seiten dieser Zahnstange zusammenwirken.

## Claims

1. Injection device which is suitable for administering a plurality of injection doses,
comprising a housing (15; 132, 150),
comprising a container (12; 136) for an injection liquid which is displaceable relative to this housing between a distal end position (fig. 2, 3, 9, 21) and a proximal end position (fig. 1, 4, 5, 8, 11, 19, 20), or a retainer (134, 234) for such a container,
comprising a push-rod (14; 188) which is moveable relative to the housing for expressing liquid from such a container (12; 136),
and comprising a first coupling device (27; 112, 114;
242, 244, 246, 248) which is provided between the push-rod (14; 188) and the container (12; 136), or a retainer (134, 234) for this container, and, during a movement of the push-rod (14; 188) in the distal direction, is designed to produce a unidirectional drive connection between push-rod (14; 188) and container (12; 136), or a retainer (134, 234) for the container, and to move this or these together with the push-rod (14; 188) in the distal direction,
wherein this first coupling device is designed to convert a movement of the push-rod (14; 188) in the proximal direction during a predetermined part (S) of this movement into a corresponding movement of the container (12; 136), or of a retainer (134, 234) for the container, in the proximal direction by means of a mechanical connection, effective during this part (S), between push-rod (14; 188) and container (12; 136), or between push-rod and a retainer (134, 234) for this container.

2. Injection device according to claim 1, wherein the unidirectional drive connection is designed so that a stop (18, 20; 70, 73; 260) for the distal movement of the container (12; 136), or of a retainer (134, 234) for the container, also forms a limit for the distal movement of the push-rod (14; 188).

3. Injection device according to claim 1 or 2, wherein the first coupling device (27; 112, 114; 242, 244, 246, 248) is designed so that it is effective both in the proximal and in the distal direction in the distal end position of the container (12; 136), or of a retainer (134, 234) for the container,
and so that it is only effective during movement of the push-rod (14; 188) in the distal direction in the proximal end position of the container (12; 136), or of a retainer for the container.

4. Injection device according to one or more of claims 1 to 3, wherein a drive member (35; 186) is provided for the push-rod (14; 188),
and a second coupling device (38; 122, 124; 214, 216, 218, 220) effective dependent on travel and/or direction is provided between the drive member (35; 186) and the push-rod (14; 188).

5. Injection device according to claim 4, wherein the second coupling device (38; 122, 124; 214, 216, 218, 220), during a movement of the drive member (35; 186) in the proximal direction, is designed to produce a unidirectional drive connection between drive member (35; 186) and push-rod (14; 188) and to move the push-rod together with the drive member (35; 186) in the proximal direction,
and to convert a movement of the drive member (35; 186) in the distal direction into a corresponding movement of the push-rod (14; 188) in the distal direction as long as the push-rod (14; 188) is not prevented from moving in the distal direction.

6. Injection device according to claim 4 or 5, wherein the second coupling device (38) produces a connection between drive member (35) and push-rod (14) both in the proximal and in the distal direction in a proximal position range (fig. 1) of the container (12).

7. Injection device according to one or more of claims 4 to 6, wherein in a distal position range (fig. 2, 3, 21) of the container (12; 136) the second coupling device (38; 214, 216, 218, 220) produces a connection between drive member (35; 186) and push-rod (14; 188) which is effective in the proximal direction and allows displacement of the drive member (35; 186) relative to the push-rod (14; 188) in the distal direction.

8. Injection device according to one or more of claims 4 to 7, wherein the drive member (35; 186) has associated therewith a spring (11; 262) acting on it in the proximal direction, in particular a spring made of plastic material, and a detent member (46; 94; 224), which spring (11; 262) can be cocked by displacement of the detent member (46; 94; 224) in the distal direction, the detent member engaging on reaching a predetermined cocked position (fig. 3, 9, 21).

9. Injection device according to claim 8, wherein a clip (98; 176) provided on the outside of the housing (15; 150) is designed to trigger the detent member (94; 224) and hence an injection operation.

10. Injection device according to one or more of claims 4 to 9, wherein the drive member (35; 186) has an actuating member (80; 206) associated therewith which is preferably located in the distal area of the device and permits displacement of the drive member (35; 186) into its cocked position (fig. 9, 21).

11. Injection device according to one or more of claims 4 to 10, wherein the unidirectional drive connection between push-rod (14; 188) and container (12; 136) and/or between drive member (35; 186) and push-rod (14; 188) is designed in the manner of a drive coupling acting in one direction, in particular in the manner of a ratchet coupling.

12. Injection device according to claim 11, wherein the coupling device is designed as a frictional coupling device and/or as a positive coupling device controlled according to travel, in the opposite direction to the unidirectional drive connection.

13. Injection device according to one or more of the preceding claims, wherein the first coupling device comprises a member (27; 112; 242, 244) connected to the container (12; 136) and designed to engage with the push-rod (14; 188).

14. Injection device according to claim 13, wherein the member (17; 112; 242, 244) connected to the container (12; 136) is in resilient engagement with the push-rod (14; 188) at least in the proximal end position of the container (12; 136).

15. Injection device according to claim 13 or 14,
wherein the container (12; 136) has a retainer (17; 54; 134, 234) associated therewith which is designed so as to be displaceable relative to the housing (15; 132, 150), the member (27; 112; 242, 244) designed for engagement with the push-rod (14; 188) being located on this retainer (17; 54, 134, 234) and displaceable with it.

16. Injection device according to one or more of the preceding claims, wherein the second coupling device comprises a member (38; 112; 186) which is provided on the drive member (35; 186) and is designed for engagement with the push-rod (14; 188) and is in resilient engagement with the push-rod (14; 188) at least in the distal end position range of the container (12; 136).

17. Injection device according to one or more of claims 13 to 16, wherein the member (27; 38) designed for engagement with the push-rod (14) is prevented from moving resiliently away from the push-rod (14) by a member or the like immovable relative to the housing and in dependence upon the position of the container (12), or of a retainer (134, 234) for this container.

18. Injection device according to one or more of the preceding claims, wherein the push-rod (14; 188) is designed at least in part in the manner of a toothed rod (fig. 10; 30, 31).

19. Injection device according to claim 18, wherein the teeth (28; 218, 248) of the toothed rod (14; 188) are designed so that they only allow movement of the toothed rod in the proximal direction relative to a resilient detent member.

20. Injection device according to one or more of the preceding claims, wherein the push-rod (14; 188) has a stop (91) associated therewith which limits at least its proximal movement relative to the housing (15; 132).

21. Injection device according to one or more of the preceding claims, wherein the container (12; 136) has a retainer (17; 54; 134, 234) associated therewith which is designed so as to be displaceable axially in the housing (15; 132) between a proximal and a distal end position and to receive a container (12; 136).

22. infection device according to claim 21, wherein the length of the retainer (54; 134, 234) is variable.

23. Injection device according to claim 22, wherein the retainer (54; 134, 234) comprises a proximal section (56; 134) and a distal section (66; 234) which are connected to one another by an adjustable connection (64; 236, 240) which allows alteration and in particular shortening of the overall length of the retainer (54; 134, 234) by means of an axial force for example (fig. 8A: K), in particular through a microdetent.

24. Injection device according to one or more of claims 21 to 23, wherein in its proximal end position the retainer (54; 134, 234) bears with its distal end against a proximal end section of the drive member.

25. Injection device according to one or more of the preceding claims, wherein the effectiveness of the first and/or the second coupling device (27; 38; 112, 114; 122, 124; 242, 244, 246, 248, 214, 216, 218, 220) is a function of the respective position of the container (12; 136).

26. Injection device according to one or more of claims 21 to 24, wherein in its distal region the retainer (54; 134, 234) is provided with stop means (68, 73; 236) which determine its proximal and/or its distal end position relative to the housing (15; 132),
and wherein the adjustment of the length of the retainer (54; 134, 234) is carried out relative to these stop means so that the postilion of the container (12; 136) relative to the push-rod (14; 188) is altered when the length of the retainer (54; 134, 234) is changed.

27. Injection device according to claim 26, wherein the device for adjustment of the length comprises a microdetent (64; 236, 240) which connects a proximal section (56; 134) and a distal section (66; 234) of the retainer (54) to one another in an adjustable manner.

28. Injection device according to one or more of the preceding claims, wherein the housing is designed in at least two parts, and a device (144) is provided for changing the relative position of housing parts (132, 150), in particular through linear relative displacement between these.

29. Injection device according to claim 28, wherein a first housing part (132) is provided for guidance of the retainer (134, 234) receiving the container (136) for the injection liquid, and a second housing part (150) displaceable relative to the first one comprises detent means (180) for detention of a detent member (224) provided on the drive member (186).

30. Injection device according to claim 29, wherein a clip (176) is provided on the outside of the second housing part (150) for releasing the detent between the detent means (180) and the detent member (224).

31. Injection device according to one or more of claims 28 to 30, wherein a thread (148, 148') is provided on one of the housing parts (150) displaceable relative to one another and a threaded sleeve (144) is provided on the other housing part (132), said threaded sleeve being rotatable relative to the latter but not displaceable axially relative to it and being in engagement with this thread (148, 148').

32. Injection device according to one or more of claims 28 to 31, wherein guiding means (152, 156, 196, 198) are provided for linear guidance between the housing parts (132, 150) displaceable relative to one another.

33. Injection device according to one or more of claims 28 to 32, wherein one of the housing parts (150) is guided in the other (132).

34. Injection device according to claim 33, wherein the outer housing part (132) is provided with at least one elongated recess (158) through which a portion (196) of the inner housing part (150) projects radially outwards.

35. Injection device according to claim 34, wherein the portion (196) of the inner housing part (150) projecting outwards is provided with an external thread (148, 148') which is in engagement with the internal thread (146) of a threaded sleeve (144) which is arranged so as to be rotatable on the outer housing part (132) but not displaceable axially relative thereto.

36. Injection device according to claim 31 or 35,
wherein the threads (146, 148, 148') are embodied as fine threads.

37. Injection device according to one or more of the preceding claims, wherein the first coupling device (186) comprises two engagement members (214, 216) which are provided with engagement elements (214', 216') which are designed for engagement in corresponding rows of teeth (218, 220) of a toothed rod (188) serving as push-rod.

38. Injection device according to one or more of the preceding claims, wherein the second coupling device comprises two engagement members (242, 244) which are provided with engagement elements (242', 244') which are designed for engagement in corresponding rows of teeth (246, 248) of a toothed rod (188) serving as push-rod.

39. Injection device according to claims 37 and 38, wherein the toothed rod (188) serving as push-rod comprises a rectangular and preferably square cross-section and is provided with rows of teeth (218, 220) for the first coupling device on two first opposite sides and with rows of teeth (246, 248) for the second coupling device on the other two opposite sides.

40. Injection device according to claim 39, wherein the engagement members (214, 216) of the first coupling device and the engagement members (242, 244) of the second coupling device engage in one another interdigitally.

41. Injection device according to one or more of claims 37 to 40, wherein the rows of teeth (218, 220) on the opposite sides of a toothed rod (188) serving as push-rod and/or the engagement elements (214', 216') on engagement members (214, 216) in resilient engagement with these opposite rows of teeth (218, 220) are offset relative to one another in the axial direction in order to allow alternatively full engagement of one engagement element of the first of two resilient engagement members with the associated row of teeth of the push-rod, or full engagement of one engagement element of the second of two resilient engagement members with the associated row of teeth of the push-rod (188) and hence refined adjustment of the injection device.

42. Injection device according to one or more of claims 37 to 41, wherein the toothed rod (188), viewed in cross-section, is embodied as a polygon and is provided in each case with a row of teeth (218, 220, 246, 248) on a plurality of sides of this polygon, which rows of teeth co-operate with a counterpart member, in particular an actuating member, which co-operates with the toothed rod (188) and is displaceable axially, which counterpart member comprises a plurality of engagement members (214, 216) which are each provided with at least one engagement element (214', 216') complementary to the associated row of teeth and through this engagement element are in resilient engagement with the associated row of teeth,
in each case one engagement member of this plurality of engagement members (214, 216) being in full engagement with the associated row of teeth (218, 220) by means of its at least one complementary engagement element (214'; 216'),
and other engagement members of this plurality of engagement members not being in full engagement with their associated row of teeth.

43. Injection device according to claim 42, wherein the rows of teeth (218, 220) of the toothed rod (188) cooperating with the counterpart member are arranged essentially symmetrical to the longitudinal axis of the toothed rod (188).

44. Injection device according to claim 42 or 43,
wherein the counterpart member is embodied as a drive member (186) for displacement of the container (136) in the housing (132, 150) of the injection device.

45. Injection device according to one or more of claims 42 to 44, wherein the toothed rod (188) comprises a rectangular, in particular square cross-section,
and the counterpart member comprises two engagement members (214, 216) which co-operate with rows of teeth (218, 220) on opposing sides of this toothed rod.

## Revendications

1. Dispositif d'injection qui est destiné à délivrer plusieurs doses, comprenant un boîtier (15 ; 132 ; 150), un réservoir (12 ; 136) pour un liquide injectable qui est mobile par rapport à ce boîtier entre une position d'extrémité distale (Fig. 2, 3, 9, 21) et une position d'extrémité proximale (Fig. 1, 4, 5, 8, 11, 19, 20), ou un support (134, 234) pour un tel réservoir, comprenant également un poussoir (14 ; 188) mobile par rapport au boîtier pour expulser du liquide d'un tel réservoir (12 ; 136), ainsi qu'un premier dispositif de liaison (27 ; 112, 114 ; 242, 244, 246, 248) prévu entre le poussoir (14 ; 188) et le réservoir (12 ; 136) ou un support (134, 234) pour ce réservoir, qui est réalisé de manière à créer entre le poussoir (14 ; 188) et le réservoir (12 ; 136) ou un support (134, 234) pour le réservoir, lors d'un déplacement du poussoir (14 ; 188) dans le sens distal, une liaison forcée unidirectionnelle, et à déplacer le réservoir et/ou le support ensemble avec le poussoir (14 ; 188) dans le sens distal, ce premier dispositif de liaison étant conçu de manière à transformer un déplacement du poussoir (14 ; 188) dans le sens proximal sur un tronçon prédéfini (S) dudit déplacement en un déplacement correspondant du réservoir (12 ; 136) ou d'un support (134, 234) pour le réservoir dans le sens proximal, au moyen d'une liaison mécanique entre le poussoir (14 ; 188) et le réservoir (12 ; 136) ou entre le poussoir et un support (134, 234) pour ledit réservoir, cette liaison mécanique étant active sur ledit tronçon (S).

2. Dispositif d'injection selon la revendication 1, dans lequel la liaison forcée unidirectionnelle est réalisée de telle sorte qu'une butée (18, 20 ; 70, 73 ; 260) pour le déplacement distal du réservoir (12 ; 136) ou d'un support (134, 234) pour ledit réservoir constitue également une limite pour le déplacement distal du poussoir (14 ; 188).

3. Dispositif d'injection selon la revendication 1 ou 2, dans lequel le premier dispositif de liaison (27 ; 112, 114 ; 242, 244, 246, 248) est réalisé de telle manière que, lorsque le réservoir (12 ; 136) ou un support (134, 234) pour le réservoir est en position d'extrémité distale, il agit aussi bien dans le sens proximal que distal et que, lorsque le réservoir (12 ; 136) ou un support pour le réservoir est en position d'extrémité proximale, il n'agit que lors d'un déplacement du poussoir (14 ; 188) dans le sens distal.

4. Dispositif d'injection selon l'une ou plusieurs des revendications 1 à 3, dans lequel est prévu un organe d'actionnement (35 ; 186) pour le poussoir (14 ; 188), et un second dispositif de liaison (38 ; 122, 124 ; 214, 216, 218, 220) est prévu entre l'organe d'actionnement (35 ; 186) et le poussoir (14 ; 188), ce second dispositif de liaison étant effectif en fonction de la position et/ou du sens de mouvement.

5. Dispositif d'injection selon la revendication 4, dans lequel le second dispositif de liaison (38 ; 122, 124 ; 214, 216, 218, 220) est réalisé de manière à créer, lors d'un déplacement de l'organe d'actionnement (35 ; 186) dans le sens proximal, une liaison forcée unidirectionnelle entre l'organe d'actionnement (35 ; 186) et le poussoir (14 ; 188), pour déplacer ce dernier ensemble avec l'organe d'actionnement (35 ; 186) dans le sens proximal, et à transformer un déplacement de l'organe d'actionnement (35 ; 186) dans le sens distal en un déplacement correspondant du poussoir (14 ; 188) dans le sens distal aussi longtemps que le poussoir (14 ; 188) n'est pas empêché de se déplacer dans le sens distal.

6. Dispositif d'injection selon la revendication 4 ou 5, dans lequel, lorsque le réservoir (12) est dans une certaine zone de positionnement proximale (fig. 1), le second dispositif de liaison (38) établit une liaison entre l'organe d'entraînement (35) et le poussoir (14) aussi bien dans le sens proximal que dans le sens distal.

7. Dispositif d'injection selon l'une ou plusieurs des revendications 4 à 6, dans lequel, lorsque le réservoir (12 ; 136) est dans une certaine zone de positionnement distale (fig. 2, 3, 21), le second dispositif de liaison (38 ; 214, 216, 218, 220) établit une liaison agissant dans le sens proximal entre l'organe d'entraînement (35 ; 186) et le poussoir (14 ; 188), laquelle liaison permet de déplacer l'organe d'entraînement (35 ; 186) par rapport au poussoir (14 ; 188) dans le sens distal.

8. Dispositif d'injection selon l'une ou plusieurs des revendications 4 à 7, dans lequel un ressort (11 ; 262) agissant sur l'organe d'entraînement (35 ; 186) dans le sens proximal, notamment un ressort en matière plastique, et un organe d'enclenchement (46 ; 94 ; 224) sont associés à l'organe d'entraînement (35 ; 186), ce ressort (11 ; 262) pouvant être tendu en déplaçant l'organe d'enclenchement (46 ; 94 ; 224) dans le sens distal, l'organe d'enclenchement s'enclenchant lorsqu'une position de tension prédéfinie est atteinte (fig. 3, 9, 21).

9. Dispositif d'injection selon la revendication 8, dans lequel un clip (98, 176) prévu sur le côté extérieur du boîtier (15 ; 150) est réalisé de manière à déclencher l'organe d'enclenchement (94 ; 224) et donc une injection.

10. Dispositif d'injection selon l'une ou plusieurs des revendications 4 à 9, dans lequel un organe d'actionnement (80 ; 206) est associé à l'organe d'entraînement (35 ; 186), cet organe d'actionnement étant de préférence situé dans la partie distale du dispositif et permettant de déplacer l'organe d'entraînement (35 ; 186) en position tendue (fig. 9, 21).

11. Dispositif d'injection selon l'une ou plusieurs des revendications 4 à 10, dans lequel la liaison forcée unidirectionnelle entre le poussoir (14 ; 188) et le réservoir (12 ; 136) et/ou entre l'organe d'entraînement (35 ; 186) et le poussoir (14 ; 188) est réalisée à la manière d'un accouplement d'entraînement agissant dans un sens, notamment à la manière d'un accouplement à cliquet.

12. Dispositif d'injection selon la revendication 11, dans lequel le dispositif de liaison dans le sens opposé à la liaison forcée unidirectionnelle est réalisé en tant que dispositif de liaison par adhérence et/ou en tant que dispositif de liaison par engagement positif commandé en fonction de la position.

13. Dispositif d'injection selon l'une ou plusieurs des revendications précédentes, dans lequel le premier dispositif de liaison présente un élément (27 ; 112 ; 242 ; 244) relié au réservoir (12 ; 136) et destiné à se mettre en prise avec le poussoir (14 ; 188).

14. Dispositif d'injection selon la revendication 13, dans lequel l'élément (17 ; 112 ; 242 ; 244) relié au réservoir (12 ; 136) est en prise élastique avec le poussoir (14 ; 188) au moins lorsque le réservoir (12 ; 136) est dans la position d'extrémité proximale.

15. Dispositif d'injection selon la revendication 13 ou 14, dans lequel un support (17 ; 54 ; 134, 234) est associé au réservoir (12 ; 136), ce support étant mobile par rapport au boîtier (15 ; 132, 150), l'élément (27 ; 112 ; 242, 244) réalisé de manière à se mettre en prise avec le poussoir (14 ; 188) étant placé sur ce support (17 ; 54 ; 134, 234) et étant mobile avec ce dernier.

16. Dispositif d'injection selon l'une ou plusieurs des revendications précédentes, dans lequel le second dispositif de liaison présente un élément (38 ; 112 ; 186) prévu sur l'organe d'entraînement (35 ; 186) et réalisé de manière à se mettre en prise avec le poussoir (14 ; 188), qui, tout au moins lorsque le réservoir (12 ; 136) est dans la zone de la position d'extrémité distale, est en prise élastique avec le poussoir (14 ; 188).

17. Dispositif d'injection selon l'une ou plusieurs des revendications 13 à 16, dans lequel, en fonction de la position du réservoir (12) ou d'un support (134, 234) pour ce dernier, l'élément (27 ; 38) réalisé de manière à se mettre en prise avec le poussoir (14) est empêché de faire un mouvement élastique pour s'éloigner du poussoir (14) par un élément fixe du boîtier ou analogue.

18. Dispositif d'injection selon l'une ou plusieurs des revendications précédentes, dans lequel le poussoir (14 ; 188) est réalisé au moins dans certaines zones à la manière d'une crémaillère (fig. 10 ; 30, 31).

19. Dispositif d'injection selon la revendication 18, dans lequel les dents (28 ; 218, 248) de la crémaillère (14 ; 188) sont réalisées de telle sorte qu'elles ne permettent à la crémaillère de se déplacer par rapport à un organe élastique d'enclenchement que dans le sens proximal.

20. Dispositif d'injection selon l'une ou plusieurs des revendications précédentes, dans lequel une butée (91) est associée au poussoir (14 ; 188), qui limite au moins le déplacement proximal de ce dernier par rapport au boîtier (15 ; 132).

21. Dispositif d'injection selon l'une ou plusieurs des revendications précédentes, dans lequel un support (17 ; 54 ; 134, 234) est associé au réservoir (12 ; 136), ce support étant mobile axialement dans le boîtier (15 ; 132) entre une position d'extrémité proximale et une position d'extrémité distale et qui est réalisé de manière à contenir un réservoir (12 ; 136).

22. Dispositif d'injection selon la revendication 21, dans le cas duquel la longueur du support (54 ; 134, 234) peut être modifiée.

23. Dispositif d'injection selon la revendication 22, dans lequel le support (54 ; 134, 234) présente un segment proximal (56 ; 134) et un segment distal (66 ; 234) qui sont reliés entre eux par une liaison réglable (64 ; 236, 240) permettant, par exemple moyennant une force axiale (fig. 8A:K) de modifier et notamment de raccourcir la longueur totale du support (54 ; 134, 234), notamment par un dispositif à micro-crans.

24. Dispositif d'injection selon l'une ou plusieurs des revendications 21 à 23, dans le cas duquel le support (54 ; 134, 234) dans sa position d'extrémité proximale est appliqué avec son extrémité distale contre un segment d'extrémité proximal de l'organe d'entraînement.

25. Dispositif d'injection selon l'une ou plusieurs des revendications précédentes, dans lequel l'efficacité du premier et/ou du second dispositif de liaison (27 ; 38 ; 112, 114; 122, 124 ; 242, 244, 246, 248, 214, 216, 218, 220) est fonction de la position du réservoir (12 ; 136).

26. Dispositif d'injection selon l'une ou plusieurs des revendications 21 à 24, dans lequel le support (54 ; 134, 234) est muni, dans sa partie distale, de moyens de butée (68, 73 ; 236) qui déterminent sa position d'extrémité proximale et/ou distale par rapport au boîtier (15 ; 132), et dans lequel la modification de longueur du support (54 ; 134, 234) s'effectue par rapport à ces moyens de butée de manière à ce que, lorsque la longueur du support (54 ; 134, 234) est modifiée, la position du réservoir (12 ; 136) par rapport au poussoir (14 ; 188) est modifiée.

27. Dispositif d'injection selon la revendication 26, dans lequel le dispositif pour modifier la longueur comporte un dispositif à micro-crans (64 ; 236, 240) qui relie entre eux un segment proximal (56 ; 134) et un segment distal (66 ; 234) du support (54) de façon réglable.

28. Dispositif d'infection selon l'une ou plusieurs des revendications précédentes, dans lequel le boîtier est au moins réalisé en deux parties et un dispositif (144) pour modifier la position relative de parties du boîtier (132, 150), notamment par déplacement relatif linéaire entre celles-ci, est prévu.

29. Dispositif d'injection selon la revendication 28, dans lequel une première partie de boîtier (132) est prévue pour guider le support (134, 234) recevant le réservoir (136) contenant le liquide à injecter, et une seconde partie de boîtier (150) mobile par rapport à la première présente des moyens d'enclenchement (180) pour enclencher un organe d'enclenchement (224) prévu sur l'organe de commande (186).

30. Dispositif d'injection selon la revendication 29, dans lequel un clip (176) est prévu sur l'extérieur de la seconde partie de boîtier (150) pour lever l'enclenchement entre les moyens d'enclenchement (180) et l'organe d'enclenchement (224).

31. Dispositif d'injection selon l'une ou plusieurs des revendications 28 à 30, dans lequel un filetage (148, 148') est prévu sur l'une des parties de boîtier (150) mobiles l'une par rapport à l'autre et une douille taraudée (144) est prévue sur l'autre partie de boîtier (132) pouvant tourner par rapport à la précédente, mais fixe axialement par rapport à celle-ci, laquelle douille est en prise avec ledit filetage (148, 148').

32. Dispositif d'injection selon l'une ou plusieurs des revendications 28 à 31, dans lequel des moyens de guidage (152, 156, 196, 198) destinés au guidage linéaire sont prévus entre les parties de boîtier (132, 150) mobiles l'une par rapport à l'autre.

33. Dispositif d'injection selon l'une ou plusieurs des revendications 28 à 32, dans lequel l'une des parties de boîtier (150) est guidée dans l'autre (132).

34. Dispositif d'injection selon la revendication 33, dans lequel la partie de boîtier extérieure (132) est munie d'au moins un évidement allongé (158) à travers lequel un segment (196) de la partie de boîtier intérieure (150) fait saillie radialement vers l'extérieur.

35. Dispositif d'injection selon la revendication 34, dans lequel la partie (196) de la partie de boîtier intérieure (150) qui fait saillie vers l'extérieur est munie d'un filetage (148, 148') qui est en prise avec le taraudage (146) d'une douille taraudée (144) placée sur la partie de boîtier extérieure (132) de manière à pouvoir tourner, mais fixe axialement.

36. Dispositif d'injection selon la revendication 31 ou 35, dans lequel les filets de vis (146, 148, 148') sont réalisés en tant que filets fins.

37. Dispositif d'injection selon l'une ou plusieurs des revendications précédentes, dans lequel le premier dispositif de liaison (186) présente deux organes d'engrènement (214, 216) qui sont munis d'éléments d'engrènement (214', 216') réalisés de manière à engrener avec des rangées de dents (218, 220) correspondantes d'une crémaillère (188) faisant office de poussoir.

38. Dispositif d'injection selon l'une ou plusieurs des revendications précédentes, dans lequel le second dispositif de liaison présente deux organes d'engrènement (242, 244) qui sont munis d'éléments d'engrènement (242', 244') réalisés de manière à se mettre en prise avec des rangées de dents (246, 248) correspondantes d'une crémaillère (188) faisant office de poussoir.

39. Dispositif d'injection selon les revendications 37 et 38, dans lequel la crémaillère (188) faisant office de poussoir présente une section transversale de préférence carrée et est munie sur deux premiers côtés opposés de rangées de dents (218, 220) pour le premier dispositif de liaison et sur les deux autres côtés opposés, de rangées de dents (246, 248) pour le second dispositif de liaison.

40. Dispositif d'injection selon la revendication 39, dans lequel les organes d'engrènement (214,216) du premier dispositif de liaison et les éléments d'engrènement (242, 244) du second dispositif de liaison s'engrènent de manière interdigitale.

41. Dispositif d'injection selon l'une ou plusieurs des revendications 37 à 40, dans lequel les rangées de dents (218, 220) sur les côtés opposés d'une crémaillère (188) faisant office de poussoir et/ou les éléments d'engrènement (214', 216') situés sur des organes d'engrènement (214, 216) engrenant élastiquement avec ces rangées de dents opposées (218, 220) sont décalés axialement les uns par rapport aux autres, afin de permettre soit à un élément d'engrènement du premier de deux organes d'engrènement élastiques d'engrener pleinement avec la rangée de dents du poussoir qui lui est associée, soit à un élément d'engrènement du second de deux organes d'engrènement élastiques d'engrener pleinement avec la rangée de dents du poussoir (188) qui lui est associée, et donc d'affiner la possibilité de réglage du dispositif d'injection.

42. Dispositif d'injection selon l'une ou plusieurs des revendications 37 à 41, dans lequel la crémaillère (188) vue en coupe transversale est réalisée en tant que polygone et est munie sur une pluralité des côtés de ce polygone d'une rangée de dents (218, 220, 246, 248), lesquelles rangées de dents coopèrent avec un organe complémentaire déplaçable axialement et coopérant avec la crémaillère (188), notamment un organe d'actionnement, lequel organe complémentaire possède plusieurs organes d'engrènement (214, 216) qui sont chacun munis d'au moins un élément d'engrènement (214', 216') complémentaire à la rangée de dents qui lui est associée et engrènent élastiquement par le biais de cet élément d'engrènement avec la rangée de dents associée, un organe d'engrènement parmi cette pluralité d'organes d'engrènement (214, 216) engrenant pleinement avec la rangée de dents associée (218, 220) par l'intermédiaire de son au moins un élément d'engrènement complémentaire (214', 216'), et d'autres organes d'engrènement parmi cette pluralité d'organes d'engrènement n'engrenant pas pleinement avec la rangée de dents qui leur est associée.

43. Dispositif d'injection selon la revendication 42, dans lequel les rangées de dents (218, 220) de la crémaillère (188) coopérant avec un organe complémentaire sont disposées essentiellement de façon symétrique par rapport à l'axe longitudinal de la crémaillère (188).

44. Dispositif d'injection selon la revendication 42 ou 43, dans lequel l'organe complémentaire est réalisé en tant qu'organe d'entraînement (186) pour déplacer le réservoir (136) dans le boîtier (132, 150) du dispositif d'injection.

45. Dispositif d'injection selon l'une ou plusieurs des revendications 42 à 44, dans lequel la crémaillère (188) présente une section transversale rectangulaire, notamment carrée, et l'organe complémentaire présente deux organes d'engrènement (214, 216) qui coopèrent avec des rangées de dents (218, 220) situées sur des côtés opposés de cette crémaillère.
